# EUROPEAN PATENT APPLICATION

(11) **EP 4 624 473 A1**
(43) Date of publication of application: **01.10.2025**
(21) Application number: 23900030.0
(22) Date of filing: 07.12.2023
(51) Int. Cl.: C07D 487/04, A61K 31/519, A61P 9/10

(54) **PYRROLO[3,2-D]PYRIMIDIN-4-ONE DERIVATIVE, AND PREPARATION METHOD THEREFOR AND MEDICAL USE THEREOF**

(30) Priority: 09.12.2022 CN 202211586176; 18.01.2023 CN 202310118965; 06.07.2023 CN 202310830294
(71) Applicant: Shenzhen Salubris Pharmaceuticals Co., Ltd., Shenzhen, Guangdong 518017 (CN)
(72) Inventor: LAI, Yingjie, Shenzhen, Guangdong 518017 (CN); WANG, Bin, Shenzhen, Guangdong 518017 (CN); YUE, Xiaoliang, Shenzhen, Guangdong 518017 (CN); WU, Kang, Shenzhen, Guangdong 518017 (CN); XIONG, Xiaolin, Shenzhen, Guangdong 518017 (CN); WEI, Qingzhu, Shenzhen, Guangdong 518017 (CN); XING, Wei, Shenzhen, Guangdong 518017 (CN)
(74) Representative: Groth & Co. KB
(86) International application number: PCT/CN2023/136923
(87) International publication number: WO 2024/120457

(57) **Abstract**

A compound of formula (I), and a preparation method therefor and a use thereof in medicine. Specifically, the present invention provides a compound of formula (I) or a stereoisomer, tautomer or pharmaceutically acceptable salt thereof, and also relates to a pharmaceutical composition containing the compound and a use of the compound in the preparation of a drug for treating and/or preventing cardiovascular diseases. The compound is an inhibitor for myeloperoxidase (MPO).

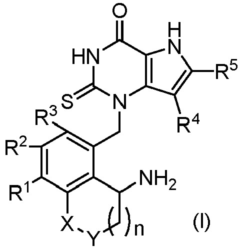

## Description

### TECHNICAL FIELD

The present disclosure belongs to the technical field of chemical drugs, and provides a series of inhibitors of myeloperoxidase (MPO). The present disclosure further relates to a pharmaceutical composition containing a compound and use of the compound in a drug for treating cardiovascular diseases.

### BACKGROUND

Myeloperoxidase (MPO) is a highly sensitive and specific indicator of vascular inflammatory mediators, and has become an important indicator for predicting the risk of cardiovascular and cerebrovascular events. MPO can catalyze oxidation of chloride ions to produce hypochlorous acid, which can kill microorganisms in phagocytic cells, destroy various target substances, and play roles in multiple aspects such as production and regulation of inflammatory reactions in the body. More importantly, MPO-oxidized low density lipoprotein (LDL) can cause atherosclerosis, so the MPO is considered to be related to occurrence of cardiovascular diseases.

At present, the MPO is considered the most promising cardiovascular marker. An elevated level of the MPO in the body indicates the risk of arteriosclerosis and coronary heart disease, and is an early warning of myocardial infarction. The MPO level is more sensitive than other indicators such as troponin T, CK-MB, and CRP, and can diagnose and assess risks earlier. The MPO level can significantly increase within 2 hours of occurrence of chest pain, so for chest pain patients, the MPO has more important clinical significance in diagnosing acute coronary syndrome (ACS).

Therefore, the present disclosure aims to discover a new MPO inhibitor, particularly a new compound with good biological properties that can be safely applied to the human body. The present disclosure also provides necessary tools for preventing and/or treating related diseases, especially cardiovascular related diseases.

### SUMMARY OF THE INVENTION

The present disclosure provides a series of pyrrolo[3,2-d]pyrimidin-4-one derivatives, and a preparation method therefor and medical use thereof.

Specifically, the present disclosure provides a compound of Formula (I) or a stereoisomer, tautomer or pharmaceutically acceptable salt thereof, wherein all variables are as defined herein.

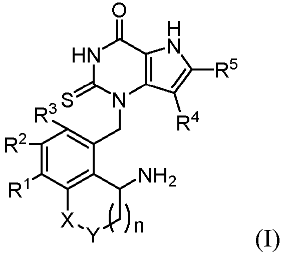

The compound is an inhibitor for myeloperoxidase (MPO). The present disclosure further relates to a pharmaceutical composition containing the compound and use of the compound in a drug for preventing and/or treating cardiovascular related diseases.

Specifically, the present disclosure is implemented through the following technical solutions:
a compound of formula (I): 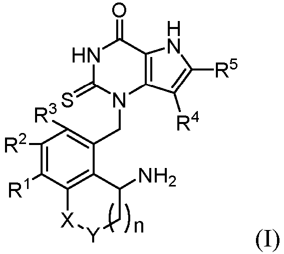
R¹, R², R³, R⁴ and R⁵ are independently selected from hydrogen, halogen, hydroxyl, amino, cyano, C₁₋₆ alkyl, C₁₋₆ alkoxyl, alkoxyalkyl, cycloalkyl, aryl or heteroaryl, wherein aryl and heteroaryl may be optionally substituted by one or more groups independently selected from halogen, hydroxyl, cyano, carboxyl, C₁₋₆ alkyl, C₁₋₆ alkoxyl, and halogenated C₁₋₆ alkyl;
X and Y are independently selected from O, CH₂ and NR⁶, and R⁶ is independently selected from hydrogen, halogen, C₁₋₆ alkyl, C₂₋₆ alkenyl, C₁₋₆ alkoxyl, alkoxyalkyl, cycloalkyl, hydroxyl, amino, cyano, cyano-substituted C₁₋₆ alkyl, amino-substituted C₁₋₆ alkyl, halogenated C₁₋₆ alkyl, halogenated C₁₋₆ alkoxyl, -(CH₂)ₙ-C(=O)-R⁷, -(CH₂)ₙ-C(= O)OR⁸, and -(CH₂)ₙ-NHC(=O)-R⁹; R⁷, R⁸ and R⁹ are independently selected from hydrogen and C₁₋₆ alkyl; and
n is 0, 1, 2 or 3.

As a preferred solution of the present disclosure, the compound is represented by formula (II): 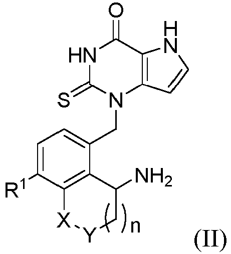 wherein, X, Y, R¹, and n are defined as above.

As a preferred solution of the present disclosure, the alkyl refers to a branched, unbranched, and cyclic saturated hydrocarbon chain containing a specified number of carbon atoms, preferably C₁₋₆ alkyl, and the C₁₋₆ alkyl is selected from methyl, ethyl, propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl.

As a preferred of the present disclosure, the C₂₋₆ alkenyl is selected from vinyl, 1-propenyl, 2-propenyl, 1-methylvinyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, and 1-ethyl-2-methyl-2-propenyl.

As a preferred solution of the present disclosure, the alkoxyl refers to an alkyl ether radical, preferably C₁₋₆ alkoxyl, and the C₁₋₆ alkoxyl is selected from methoxy, ethoxy, propoxy, isopropoxy, n-butoxy, isobutoxy, sec-butoxy, tert-butoxy, n-pentyloxy, sec-pentyloxy, 1-ethylpropoxy, 2-methylbutoxy, tert-pentyloxy, 1,2-dimethylpropoxy, isopentyloxy, neopentyloxy, n-hexyloxy, isohexyloxy, sec-hexyloxy, tert-hexyloxy, neohexyloxy, 2-methylpentyloxy, 1,2-dimethylbutoxy, and 1-ethylbutoxy.

As a preferred solution of the present disclosure, the alkoxyalkyl refers to substitution of one or more hydrogen atoms of alkyl by alkoxyl, preferably C₁₋₄ alkoxyl C₁₋₄ alkyl, further selected from methoxymethyl, methoxyethyl, methoxypropyl, methoxybutyl, ethoxymethyl, ethoxyethyl, ethoxypropyl, ethoxybutyl, propoxymethyl, propoxyethyl, propoxypropyl, propoxybutyl, butoxymethyl, butoxyethyl, butoxypropyl, butoxybutyl, etc.

As a preferred solution of the present disclosure, the halogen is selected from fluorine, chlorine, bromine, and iodine, halogenated C₁₋₆ alkyl refers to substitution of one or more hydrogen atoms of C₁₋₆ alkyl by halogen, and halogenated C₁₋₆ alkoxyl refers to substitution of one or more hydrogen atoms of C₁₋₆ alkoxyl by halogen.

The cyano-substituted C₁₋₆ alkyl refers to substitution of one or more hydrogen atoms of C₁₋₆ alkyl by cyano, and the amino-substituted C₁₋₆ alkyl refers to substitution of one or more hydrogen atoms of C₁₋₆ alkyl by amino.

As a preferred solution of the present disclosure, the aryl is selected from phenyl; and the heteroaryl is selected from 5- to 12- membered heteroaryl, and the 5- to 12- membered heteroaryl is selected from

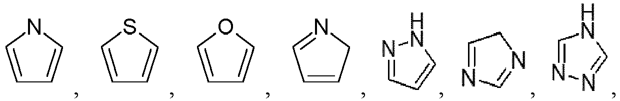

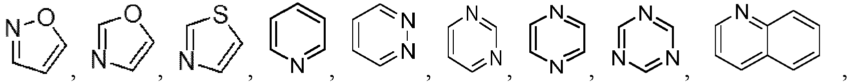

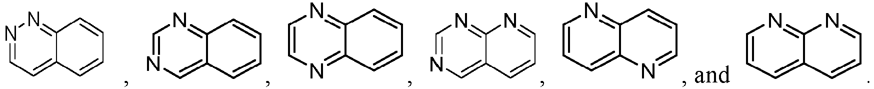

As a preferred solution of the present disclosure, the "alkylene" refers to alkyl that can be independently substituted by 0 to 2 independent substituents when a valency allows, the cyano refers to a -CN group, the hydroxyl refers to a -OH group, and sulfonyl refers to an -SO₂ group.

As a preferred solution of the present disclosure, cycloalkane is selected from C₃₋₆ cycloalkyl, and the C₃₋₆ cycloalkyl is selected from cyclopropyl, cyclobutyl, cyclopentyl, and cyclohexyl.

As a preferred solution of the present disclosure, the compound or the stereoisomer, the tautomer, or the pharmaceutically acceptable salt thereof is represented by formula (I), wherein R¹ is selected from hydrogen or chlorine;
R², R³ and R⁵ are selected from hydrogen;
R⁴ is selected from hydrogen, methyl, and methoxy;
X is O, CH₂, N-CH₃, or N-C(=O)-O-CH₂-CH₃;
Y is O, CH₂, or N-CH₃; and
N is 0, 1 or 2.

As a preferred solution of the present disclosure, the compound or the pharmaceutically acceptable salt thereof is selected from the following compounds:

| | Structural formula | | Structural formula | | Structural formula |
|---|---|---|---|---|---|
| 1 | 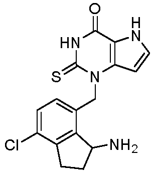 | 2 | 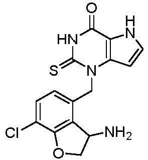 | 3 | 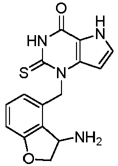 |
| 4 | 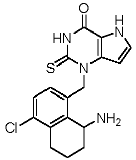 | 5 | 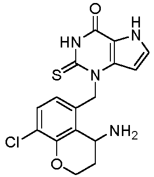 | 6 | 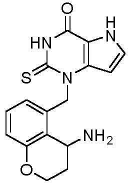 |
| 7 | 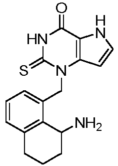 | 8 | 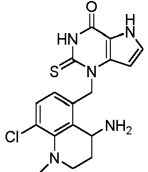 | 9 | 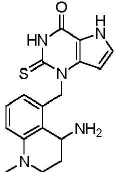 |
| 10 | 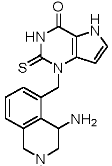 | 11 | 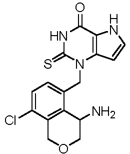 | 12 | 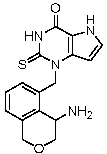 |
| 13 | 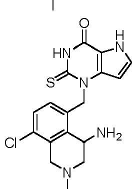 | 14 | 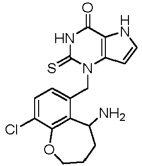 | 15 | 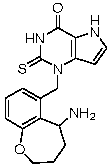 |
| 16 | 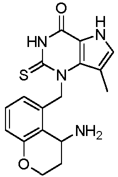 | 17 | 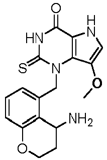 | | |

As a preferred solution of the present disclosure, the compound or the pharmaceutically acceptable salt thereof is selected from the following compounds:

| | Structural formula | | Structural formula |
|---|---|---|---|
| 1A | 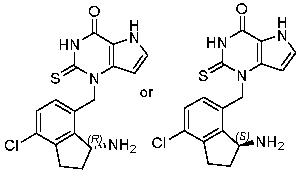 | 1B | 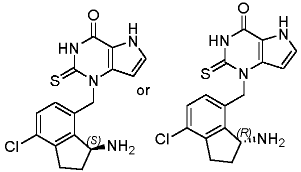 |
| 2 | 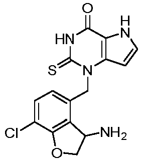 | 3 | 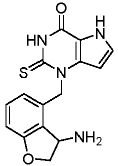 |
| 4 | 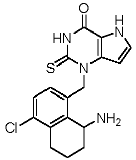 | 5 | 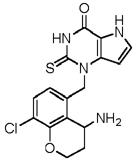 |
| 6A | 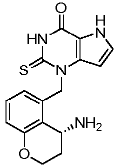 | 6B | 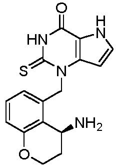 |
| 7A | 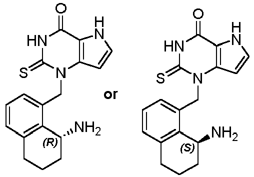 | 7B | 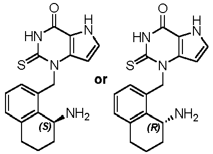 |
| 8 | 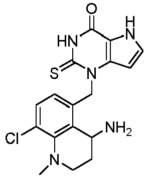 | 9 | 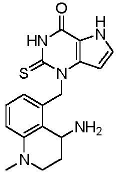 |
| 10 | 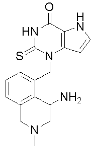 | 11 | 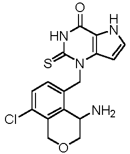 |
| 12 | 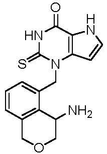 | 13 | 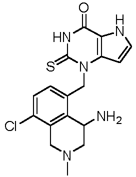 |
| 14 | 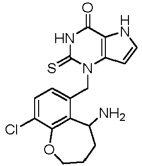 | 15A | 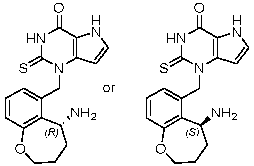 |
| 15B | 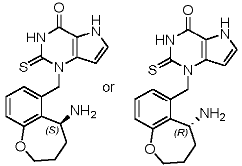 | 16 | 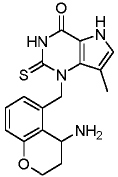 |
| 17 | 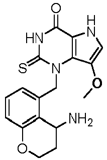 | | |

As a preferred solution of the present disclosure, the pharmaceutically acceptable salt refers to a salt prepared from a compound and a pharmaceutically acceptable acid or base.

As a preferred solution of the present disclosure, one or more hydrogen atoms of the compound are substituted by isotopes, preferably deuterium.

Another objective of the present disclosure is to provide a pharmaceutical composition, including the compound of formula (I), or the stereoisomer, the tautomer or the pharmaceutically acceptable salt thereof above, and one or more pharmaceutically acceptable carriers.

Another objective of the present disclosure is to provide medical use of the compound of formula (I), or the stereoisomer, the tautomer or the pharmaceutically acceptable salt thereof, specifically, use in preparation of a drug for treating and/or preventing MPO-related diseases, and specifically relates to use in a drug for cardiovascular related diseases.

Unless otherwise specified, the following terms and phrases used herein are intended to have the following meanings. A specific term or phrase should not be considered uncertain or unclear without a specific definition, but should be understood according to ordinary meanings. When a commodity name appears herein, it is intended to refer to a commodity or an active ingredient corresponding to the commodity name. The term "pharmaceutically acceptable" used here refers to compounds, materials, compositions, and/or dosage forms that are suitable for use in contact with human and animal tissue within the scope of reliable medical judgment, without excessive toxicity, irritation, allergic reactions, or other issues or complications, and are commensurate with a reasonable benefit/risk ratio.

The term "pharmaceutically acceptable salt" refers to a salt of the compound in the present disclosure, and is prepared from compounds with specific substituents discovered in the present disclosure and a pharmaceutically acceptable acid or base.

In addition to the salt form, the compound provided by the present disclosure also has a prodrug form. A prodrug of the compound described herein easily undergoes chemical changes under a physiological condition, thereby being transformed into the compound of the present disclosure. In addition, the prodrug can be converted into the compound of the present disclosure through a chemical or biochemical method in an in vivo environment.

Certain compounds of the present disclosure may exist in a non-solvation or solvation form, including a hydrate form. Generally speaking, the solvation form and the non-solvation form are equivalent and both contained within the scope of the present disclosure.

The compound of the present disclosure may exist in a specific geometric or stereoisomeric form. The present invention envisions all such compounds, including cis and trans isomers, (-)- and (+)- enantiomers, (R)- and (S)- enantiomers, a diastereomer, a (D)-isomer, an (L)- isomer, as well as racemic mixtures thereof and other mixtures, such as enantiomer or diastereomer enriched mixtures, all of which fall within the scope of the present disclosure. Additional asymmetric carbon atoms may exist in alkyl and other substituents. All of these isomers and their mixtures are included within the scope of the present disclosure.

Optically active (R)- and (S)- isomers, as well as D and L isomers, may be prepared through chiral synthesis or chiral reagents or other conventional techniques. If one wishes to obtain an enantiomer of a certain compound in the present invention, it may be prepared by asymmetric synthesis or by derivatization with chiral adjuvants, wherein a resulting diastereomer mixture is separated, and an auxiliary group is cleaved to provide the desired pure enantiomer. Alternatively, when the molecules contain alkaline functional groups (such as amino) or acidic functional groups (such as carboxyl), a salt of the diastereomer is formed with an appropriate optically-active acid or alkali, then diastereomer separation is performed using conventional methods known in the art, and then recovery is performed to obtain the pure enantiomer. In addition, the separation of the enantiomer and the diastereomer is typically achieved using chromatography, and the chromatography employs a chiral stationary phase and is optionally combined with a chemical derivatization method (such as generating carbamate from amine).

Atoms of molecules of the compound in the present invention are isotopes, and isotope derivatization may generally be used to prolong a half-life, reduce a clearance rate, stabilize metabolism, increase in-vivo activity and achieve other effects. Moreover, one implementation solution in which at least one atom is substituted by an atom with the same atom number (proton number) and different mass numbers (a sum of protons and neutron) is included. Examples of the isotopes included in the compound of the present disclosure include hydrogen atoms, carbon atoms, nitrogen atoms, oxygen atoms, phosphorus atoms, sulfur atoms, fluorine atoms, and chlorine atoms, which respectively include ²H, ³H, ¹³C, ¹⁴C, ¹⁵N, ¹⁷O, ¹⁸O, ³¹P, ³²P, ³⁵S, ¹⁸F, and ³⁶Cl. Particularly, radioactive isotopes such as ³H or ¹⁴C that emit radiation as they decay may be used for local anatomical examination of drug formulations or in-vivo compounds. Stable isotopes neither decay or change with their quantity, nor has radioactivity, so the stable isotopes can be safely used. When the atoms constituting the molecules of the compound of the present disclosure are the isotopes, the isotopes can be transformed according to a general method by replacing a reagent used in synthesis with a reagent containing the corresponding isotopes.

The compound of the present disclosure may contain unnatural proportions of atomic isotopes on one or more atoms constituting the compound. For example, the compound may be labeled with radioactive isotopes such as deuterium (²H), iodine-125 (¹²⁵I), or C-14 (¹⁴C). Transformations of all isotope compositions of the compound of the present disclosure, whether radioactive or not, are included within the scope of the present disclosure.

Further, the compound of the present disclosure has one or more hydrogen atoms substituted by the isotope deuterium (²H). After deuteration, the compound of the present disclosure has the effects of prolonging half-life, decreasing clearance rate, metabolic stability, improving in vivo activity, etc.

A method for preparing isotope derivatives usually includes a phase transfer catalysis method. For example, the preferred deuteration method uses a phase transfer catalyst (such as tetrabutylammonium bromide (TBAB)). Using the phase transfer catalyst to exchange a methylene proton of a diphenylmethane compound results in introduction of a higher deuterium level compared to reducing with sodium deuterated borate using deuterated silane (such as triethyldeuterated silane) in the presence of an acid (such as methanesulfonic acid) or using a Lewis acid such as aluminum trichloride.

A term "pharmaceutically acceptable carrier" refers to any preparation carrier or medium capable of delivering an effective dosage of the active substance of the present disclosure, not interfering with biological activity of the active substance, and having no toxic side effects to a host or a patient. Representative carriers include water, oil, vegetables and minerals, cream bases, lotion bases, ointment bases, etc. These bases include suspension agents, thickening agents, transdermal enhancers, etc. Their preparations are well known to those skilled in a cosmetics field or a topical drug field. For other information about the carrier, please refer to Remington: The Science and Practice of Pharmacy, 21st Ed., Lippincott, Williams & Wilkins (2005), of which the contents are incorporated herein by reference.

A term "excipient" typically refers to a carrier, a diluent, and/or a medium required for the preparation of an effective pharmaceutical composition.

For drugs or pharmacologically active agents, a term "effective dosage" or "therapeutic effective dosage" refers to a sufficient usage amount of drugs or medicament that is non-toxic but achieves the desired effect. For an oral dosage form in the present disclosure, the "effective dosage" of one active substance in the composition refers to an amount required to achieve the expected effect when combined with another active substance in the composition. The determination of the effective dosage varies from person to person, depending on an age and general situation of a receptor, as well as the specific active substance. The appropriate effective dosage in each case may be determined by those skilled in the art according to routine experiments.

Terms "active ingredient," "therapeutic agent," "active substance" or "active agent" refer to a chemical entity that can effectively treat target disorders, diseases or conditions.

"Optional" or "optionally" means that the subsequently described event or circumstance may possibly but not necessarily occur, and that the description includes an instance where the event or circumstance occurs and an instance where the event or circumstance does not occur.

The compound of the present disclosure may be prepared by various synthetic methods well-known to those skilled in the art, including the specific implementations listed below, the implementations formed by combination of the specific implementations with other chemical synthesis methods, and equivalent substitution manners well-known to those skilled in the art. Preferred implementations include but are not limited to the examples of the present disclosure.

### BRIEF DESCRIPTION OF THE DRAWINGS

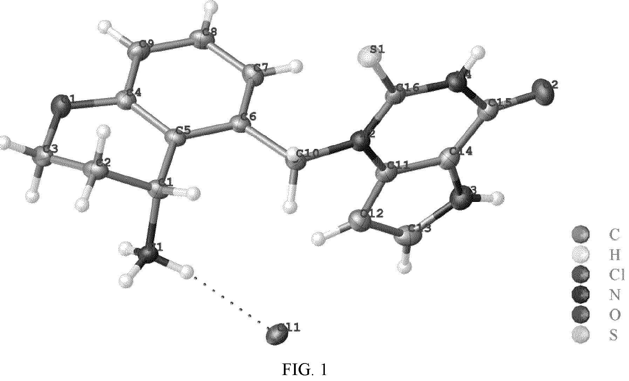

FIG. 1 is a schematic structural diagram of a single crystal of 6A hydrochloride in Example 6.

### DETAILED DESCRIPTION

The present disclosure will be further described in detail with reference to examples, but the implementations of the present disclosure are not limited to these.

A structure of a compound is determined by nuclear magnetic resonance (NMR) or mass spectrometry (MS). NMR shift (δ) is given in unit of 10⁻⁶ (ppm). The determination of the NMR is carried out using a Bruker AVANCE-III NMR instrument, with deuterated dimethyl sulfoxide (DMSO-d₆) and deuterated chloroform (CDCl₃) as solvents, and tetramethylsilane (TMS) as the internal standard.

The determination of MS is carried out using an ISQ EC mass spectrometer (manufacturer: Thermo, model: ISQ EC).

High performance liquid chromatography (HPLC) analysis is performed using Thermo U3000 HPLC DAD high performance liquid chromatograph and Agilent 1260 high performance liquid chromatograph.

CombiFlash Rf+LUMEN (TELEDYNE ISCO) is used as a CombiFlash rapid preparation instrument.

A Yantai Yinlong HSGF₂₅₄ or GF₂₅₄ silica gel plate is used as a thin layer chromatography silica gel plate. The specification of the silica gel plate used for thin layer chromatography (TLC) is in a range from 0.17 mm to 0.23 mm, and the specification of a thin layer chromatography separation and purification product is in a range from 0.4 mm to 0.5 mm.

A silica gel column chromatography generally uses 100-200 meshes of Rushan Sanpont New silica gel as a carrier.

Reagents: NaBH₃-sodium borohydride, EtOH-ethanol, NaOAc-sodium acetate, toluene, r.t.-room temperature, NaCO₃-sodium carbonate, MeOH-methanol, AcOH-acetic acid, AcONa-sodium acetate, H₂SO₄-sulfuric acid, NaOH-sodium hydroxide, CS₂CO₃-cesium carbonate, TEMPO-2,2,6,6,-tetramethylpiperidine oxide, TMSN₃-azidotrimethylsilane, ACN-acetonitrile, NaBH₃CN-cyanoborohydride, *p*-TsOH-p-toluenesulfonic acid, NaH-sodium hydride, THF-tetrahydrofuran, CH₃I-iodomethane, acetone, CCL₄-carbon tetrachloride, NBS- N-bromosuccinimide, AIBN-azobisisobutyronitrile, DIEA-*N,N*-diisopropylethylamine, DMF- *N,N*-dimethylformamide, Pd(PPh₃)₂Cl₂--bis(triphenylphosphine)palladium(II)chloride, LiCL-lithium chloride, n-BuLi-n-butyllithium, NH₂OH HCl-hydroxylamine hydrochloride, and AgNO₃-silver nitrate.

abs refers to an absolute configuration.

### Example 1

### 1-((3-amino-7-chloro-2,3-dihydro-1H-inden-4-yl)methyl)-2-thio-1,2,3,5-tetrahydro-4H-pyrrolo[3,2-d]pyrimidin-4-one, compounds 1A and 1B

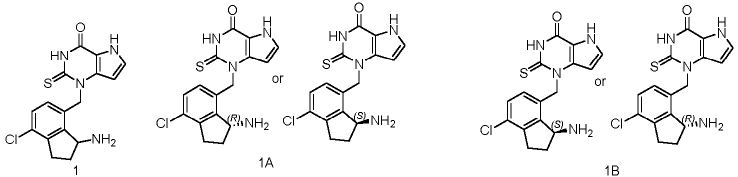

### Synthetic route:

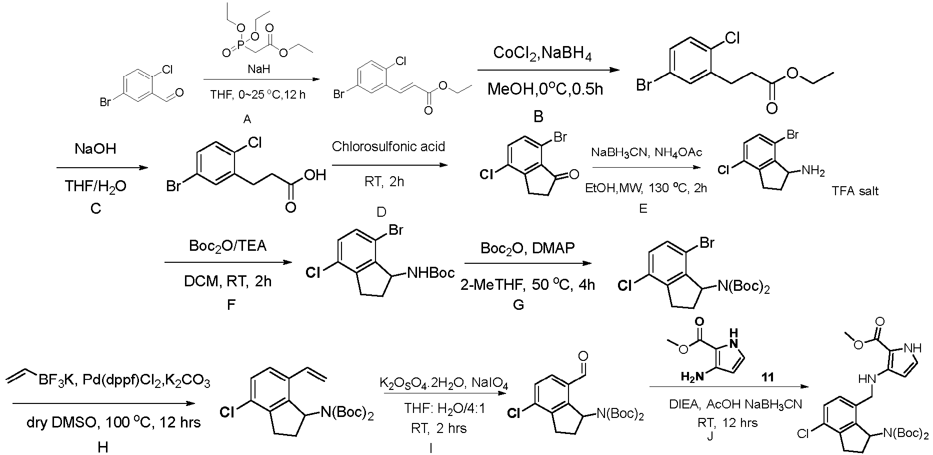

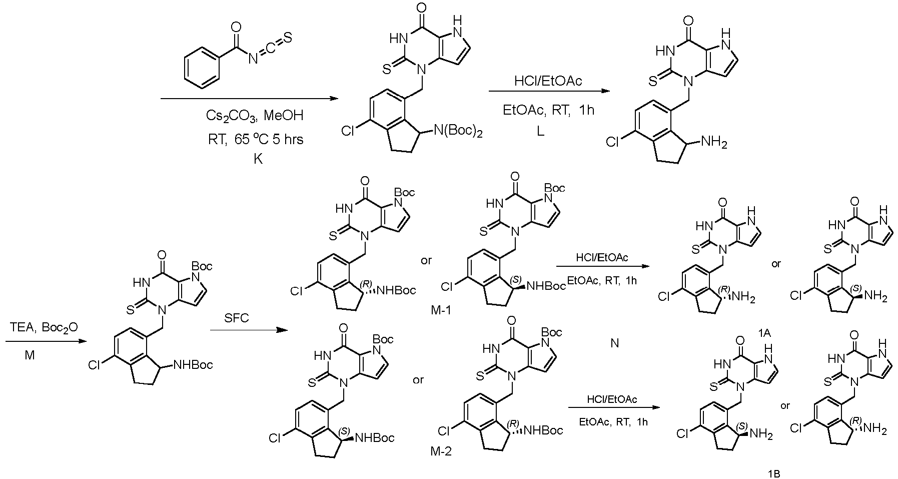

### Step A: synthesis of ethyl 3-(5-bromo-2-chlorophenyl)acrylate

2-(diethoxyphosphate)ethyl acetate (11.2 g, 50.2 mmol) was dissolved in THF (100 mL), and placed in an ice water bath, NaH (2.0 g, 50.2 mmol) was added for a reaction for 30 minutes, then 5-bromo-2-chlorobenzaldehyde (10 g, 45.6 mmol) was added, and reaction was performed at a room temperature for 12 hours.

At the end of the reaction, 30 mL of water was added to a mixture for quenching, ethyl acetate (20 mL×3) was used for extraction, and organic phases were combined, washed with a saturated saline solution, dried with anhydrous sodium sulfate, filtered, and subjected to spin drying. Without further purification, 13 g of white solid ethyl 3-(5-bromo-2-chlorophenyl)acrylate was obtained (yield: 98.5%). ¹H NMR (400 MHz, DMSO) δ 8.20 (d, J=2.4 Hz, 1H), 7.81 (d, J=16.0 Hz, 1H), 7.64 (dd, J=8.4, 2.4 Hz, 1H), 7.51 (d, J=8.4 Hz, 1H), 6.85 (d, J=16.0 Hz, 1H), 4.22 (q, J=7.0 Hz, 2H), 1.26 (q, J=6.8 Hz, 4H).

### Step B: synthesis of 2-bromo-4-chlorophenylethyl-2-methylpropane-2-sulfinamide

Ethyl 3-(5-bromo-2-chlorophenyl)acrylate (11.7 g, 40.6 mmol) was dissolved in methanol (100 mL), cobalt chloride (968 mg, 4.07 mmol) was added, sodium borohydride (2.3 g, 61.0 mmol) was added in an ice water bath, and reaction was performed at 0°C for 30 minutes.

After the reaction was complete, the methanol in the solution was subjected to spin drying, 30 mL of water was added to a mixture, ethyl acetate (20 mL×3) was used for extraction, and organic phases were combined, washed with a saturated saline solution, dried with anhydrous sodium sulfate, filtered, and subjected to spin drying. Without further purification, 11 g of green solid 2-bromo-4-chlorophenylethyl-2-methylpropane-2-sulfinamide was obtained (yield: 92.9%). ¹H NMR (400 MHz, DMSO) δ 7.58 (d, J=2.4 Hz, 1H), 7.45 (dd, J=8.5, 2.4 Hz, 1H), 7.39 (d, J=8.5 Hz, 1H), 4.15-3.95 (m, 2H), 2.94 (t, J=7.6 Hz, 2H), 2.63 (t, J=7.6 Hz, 2H), 1.21-1.10 (m, 4H).

### Step C: synthesis of 3-(5-bromo-2-chlorophenyl)propanoic acid

2-bromo-4-chlorophenylethyl-2-methylpropane-2-sulfinamide (11 g, 37.8 mmol) was dissolved in a 1:1 mixture (30 mL) of tetrahydrofuran and water, and a mixture entered sodium hydroxide (3.0 g, 75.6 mmol) dissolved in water to react at a room temperature for 16 hours.

At the end of the reaction, HCl was added to adjust a pH to 3, tetrahydrofuran was subjected to spin drying, ethyl acetate (20 mL×3) was used for extraction, organic phases were combined, washed with a saturated saline solution, dried with anhydrous sodium sulfate, filtered, and subjected to spin drying, so as to obtain 9 g of white solid 3-(5-bromo-2-chlorophenyl)propanoic acid (yield: 90.5 %). ¹H NMR (400 MHz, DMSO) δ 12.25 (s, 1H), 7.57 (d, J=2.0 Hz, 1H), 7.43 (dt, J=22.4, 5.4 Hz, 2H), 2.91 (t, J=7.6 Hz, 2H), 2.54 (dd, J=14.0, 6.4 Hz, 2H).

### Step D: synthesis of 7-bromo-4-chloro-2,3-dihydro-1H-inden-1-one

3-(5-bromo-2-chlorophenyl)propanoic acid (3.5 g, 13.3 mmol) was dissolved in dichloromethane (10 mL), chlorosulfonic acid (6 mL) was added to a reaction solution, and reaction was performed at a room temperature for 2 hours.

At the end of the reaction, the reaction was quenched with ice water, 1 mol of sodium hydroxide solution was used to adjust a pH to 10, dichloromethane (20 mL×3) was used for extraction, organic phases were combined, washed with a saturated saline solution, dried with anhydrous sodium sulfate, filtered, and subjected to spin drying. The resulting residue was purified by silica gel column chromatography (an eluent: petroleum ether/ethyl acetate=5/1) to obtain 1.2 g of 7-bromo-4-chloro-2,3-dihydro-1H-inden-1-one (yield: 36.8%). ¹H NMR (400 MHz, DMSO) δ 7.76-7.53 (m, 2H), 3.12-2.93 (m, 2H), 2.76-2.65 (m, 2H).

### Step E: synthesis of 7-bromo-4-chloro-2,3-dihydro-1H-indene-1-amine

7-bromo-4-chloro-2,3-dihydro-1H-inden-1-one (500 mg, 2.03 mmol) was dissolved in anhydrous ethanol (6 mL), ammonium acetate (2.35 g, 30.5 mmol) and sodium cyanoborohydride (153 mg, 2.44 mmol) were added for a reaction under a microwave at 130°C for 2 hours.

At the end of the reaction, ethanol in the solution was subjected to spin drying, 1 mol of sodium hydroxide solution was used to adjust a pH to 10, ethyl acetate (10 mL×3) was used for extraction, organic phases were combined, dried with anhydrous sodium sulfate, filtered, and concentrated to dryness, and the resulting residue was purified using a reversed-phase column (0.1% TFA system) to obtain 1.1 g of 7-bromo-4-chloro-2,3-dihydro-1H-indene-1-amine (yield 79.0%). ¹H NMR (400 MHz, DMSO) δ 8.28 (s, 3H), 7.57 (d, *J*=8.4 Hz, 1H), 7.45 (d, *J*=8.4 Hz, 1H), 4.81 (d, *J*=6.4 Hz, 1H), 3.25 (dt, *J*=17.0, 8.4 Hz, 1H), 3.00 (dd, *J*=16.8, 9.2, 1.6 Hz, 1H), 2.50-2.42 (m, 1H), 2.16 (dd, *J*=14.4, 7.8 Hz, 1H).

### Step F: synthesis of tert-butyl (7-bromo-4-chloro-2,3-dihydro-1H-inden-1-yl)carbamate

7-bromo-4-chloro-2,3-dihydro-1H-indene-1-amine (1.1 g, 4.5 mmol), di-tert-butyl dicarbonate (1.07 g, 4.9 mmol), and triethylamine (1.35 g, 13.4 mmol) were dissolved in dichloromethane (10 mL), and a mixture reacted at a room temperature for 2 hours.

25 mL of water was added to a reaction solution, dichloromethane (20 mL×2) was used for extraction, and organic phases were combined, washed with a NaHCO₃ aqueous solution, dried with anhydrous sodium sulfate, filtered, and subjected to spin drying. Without further purification, 1.5 g of tert-butyl (7-bromo-4-chloro-2,3-dihydro-1H-inden-1-yl)carbamate (yield: 97%) was obtained. ¹H NMR (400 MHz, CDCl₃) δ 7.31 (d, *J*=8.4 Hz, 1H), 7.12 (d, *J*=8.4 Hz, 1H), 5.27 (d, *J*=22.0 Hz, 1H), 4.68 (s, 1H), 2.94 (dd, *J*=17.0, 9.0, 4.0 Hz, 1H), 2.48 (td, *J*=16.8, 8.0 Hz, 1H), 2.12-2.04 (m, 1H), 1.46 (s, 9H).

### Step G: synthesis of tert-butyl(7-bromo-4-chloro-2,3-dihydro-1H-inden-1-yl)(tert-butoxycarbonyl)carbama te

Tert-butyl (7-bromo-4-chloro-2,3-dihydro-1H-inden-1-yl)carbamate (1.5 g, 4.32 mmol), di-tert-butyl dicarbonate (1.89 g, 8.65 mmol), and 4-dimethylaminopyridine (1.05 g, 8.65 mmol) were dissolved in dimethyltetrahydrofuran (15 mL), and a mixture reacted at 55°C for 4 hours.

At the end of the reaction, the mixture was cooled to a room temperature and subjected to spin drying, 20 mL of water was added, ethyl acetate (25 mL×2) was used for extraction, and organic phases were combined, washed with a NaHCO₃ aqueous solution, dried with anhydrous sodium sulfate, filtered, and subjected to spin drying. The resulting residue was purified using a reversed-phase column (0.1% ammonia water system) to obtain 1.4 g of tert-butyl(7-bromo-4-chloro-2,3-dihydro-1H-inden-1-yl)(tert-butoxycarbonyl)carbamate (yield: 72.5%). ¹H NMR (400 MHz, CDCl₃) δ 7.25 (s, 1H), 7.08 (t, *J*=8.8 Hz, 1H), 5.96 (dd, *J*=9.8, 6.4 Hz, 1H), 3.18 (ddd, *J*=32.4, 18.4, 13.2 Hz, 1H), 2.95-2.82 (m, 1H), 2.63-2.50 (m, 1H), 2.25-2.16 (m, 1H), 1.46-1.27 (m, 18H).

### Step H: synthesis of tert-butyl(tert-butoxycarbonyl)(4-chloro-7-vinyl-2,3-dihydro-1H-inden-1-yl)carbamate

Tert-butyl(7-bromo-4-chloro-2,3-dihydro-1H-inden-1-yl)(tert-butoxycarbonyl)carbama te (1.4 g, 3.13 mmol), potassium vinyltrifluoroborate (0.54 g, 4.07 mmol), 1,1'-bis(diphenylphosphine)ferrocene palladium chloride (0.42 g, 0.62 mmol), and potassium carbonate (1.29 g, 9.39 mmol) were dissolved in dimethyl sulfoxide (14 mL), and a mixture reacted at 100°C under nitrogen protection for 12 hours.

At the end of the reaction, the mixture was cooled to a room temperature, a reaction solution was filtered through diatomaceous earth, filtrate was collected, then 50 mL of water was added, ethyl acetate (50 mL×3) was used for extraction, and organic phases were combined, washed with a NaCl aqueous solution, dried with anhydrous sodium sulfate, filtered, and subjected to spin drying. The resulting residue was purified by silica gel column chromatography (an eluent: petroleum ether/ethyl acetate=10/1) to obtain 1.2 g of tert-butyl(tert-butoxycarbonyl)(4-chloro-7-vinyl-2,3-dihydro-1H-inden-1-yl)carbamate (yield: 97%). ¹H NMR (400 MHz, DMSO) δ 7.53-7.32 (m, 1H), 7.36-7.20 (m, 1H), 6.68 (dd, *J*=17.6, 11.2 Hz, 1H), 5.93 (dt, *J*=31.2, 15.6 Hz, 1H), 5.77 (dt, *J*=17.6, 5.2 Hz, 1H), 5.38-5.09 (m, 1H), 3.11-2.96 (m, 1H), 2.91-2.75 (m, 1H), 2.59-2.51 (m, 1H), 2.14-2.01 (m, 1H), 1.34-1.20 (m, 18H).

### Step I: synthesis of tert-butyl(tert-butoxycarbonyl)(4-chloro-7-formyl-2,3-dihydro-1H-inden-1-yl)carbama te

Tert-butyl(tert-butoxycarbonyl)(4-chloro-7-vinyl-2,3-dihydro-1H-inden-1-yl)carbamate (1.2 g, 3.04 mmol) was dissolved in a mixed solution of tetrahydrofuran (24 mL) and water (6 mL), potassium osmate (112 mg, 0.30 mmol) was added to a reaction solution, the reaction solution was stirred at a room temperature for 30 minutes, then sodium periodate (2.61 g, 12.2 mmol) was added to the reaction solution, and the reaction solution reacted at a room temperature for 2 hours.

At the end of the reaction, 20 mL of water was added to the reaction solution, ethyl acetate (20 mL×4) was used for extraction, and organic phases were combined, dried with anhydrous sodium sulfate, filtered, and subjected to spin drying. The resulting residue was purified by silica gel column chromatography (an eluent: petroleum ether/ethyl acetate=10/1) to obtain 1.0 g of tert-butyl(tert-butoxycarbonyl)(4-chloro-7-formyl-2,3-dihydro-1H-inden-1-yl)carbamate (yield: 83.3%). ¹H NMR (400 MHz, DMSO) δ 10.03 (s, 1H), 7.74 (d, *J*=8.0 Hz, 1H), 7.58 (d, *J*=8.0Hz, 1H), 6.22 (dd, *J*=9.6, 4.8 Hz, 1H), 3.16-2.99 (m, 1H), 2.95-2.82 (m, 1H), 2.69-2.50 (m, 3H), 2.13-2.02 (m, 1H), 1.30 (s, 18H).

### Step J: synthesis of methyl 3-(((3-(bis(tert-butoxycarbonyl)amino)-7-chloro-2,3-dihydro-1H-inden-4-yl)methyl)ami no)-1H-pyrrole-2-carboxylate

3-amino-2-ethoxycarbonylpyrrole hydrochloride (529 mg, 2.78 mmol) was dissolved in 10 mL of anhydrous ethanol, then N,N-diisopropylethylamine (359 mg, 2.78 mmol) and glacial acetic acid (456 mg, 7.59 mmol) were added to a mixture, the mixture was stirred at a room temperature for 10 minutes, then tert-butyl(tert-butoxycarbonyl)(4-chloro-7-formyl-2,3-dihydro-1H-inden-1-yl)carbamate (1.0 g, 2.53 mmol) was added to the mixture, and stirred at a room temperature for 2 hours, then sodium cyanoborohydride (191 mg, 3.04 mmol) was added to the mixture, and the mixture reacted at a room temperature for 12 hours.

At the end of the reaction, the anhydrous ethanol in the reaction solution was subjected to spin drying. 10 mL of water and ethyl acetate (20 mL×2) were used for extraction, and organic phases were combined, dried with anhydrous sodium sulfate, filtered, and subjected to spin drying. The resulting residue was purified using a reversed-phase column (an eluent: 0.1% ammonia water solution) to obtain 1.0 g of methyl 3-(((3-(bis(tert-butoxycarbonyl)amino)-7-chloro-2,3-dihydro-1H-inden-4-yl)methyl)amino)-1H-pyrrole-2-carboxylate (yield: 74.6%). ¹H NMR (400 MHz, DMSO) δ 10.77 (s, 1H), 7.33-7.20 (m, 1H), 7.14 (d, *J*=8.0 Hz, 1H), 6.70 (t, *J*=3.2 Hz, 1H), 5.97 (dd, *J*=9.6, 4.4 Hz, 1H), 5.77 (s, 1H), 5.44 (t, *J*=2.4 Hz, 1H), 4.26-4.09 (m, 4H), 3.12-2.98 (m, 1H), 2.91-2.75 (m, 1H), 2.56 (dd, *J*=14.0, 9.9, 5.0 Hz, 1H), 2.09 (tt, *J*=16.0, 5.2 Hz, 1H), 1.30 (s, 18H), 1.30-1.24 (m, 3H).

### Step K: synthesis of tert-butyl (tert-butoxycarbonyl)(4-chloro-7-((4-oxo-2-thio-2,3,4,5-tetrahydro-1H-pyrrolo[3,2-d]p yrimidin-1-yl)methyl)-2,3-dihydro-1H-inden-1-yl

Methyl 3-(((3-(bis(tert-butoxycarbonyl)amino)-7-chloro-2,3-dihydro-1H-inden-4-yl)methyl)amino)-1H-pyrrole-2-carboxylate (0.9 g, 1.68 mmol) and benzoyl isothiocyanate (0.32 g, 2.02 mmol) ware dissolved in methanol (5 mL), a mixture was stirred at a room temperature for 3 hours, then cesium carbonate (1.09 g, 3.36 mmol) was added, and a reaction solution reacted at 65°C for 2 hours.

At the end of the reaction, the anhydrous methanol in the reaction solution was subjected to spin drying. 20 mL of water and ethyl acetate (20 mL×2) were used for extraction, and organic phases were combined, dried with anhydrous sodium sulfate, filtered, and subjected to spin drying. The resulting residue was purified using a reversed-phase column (an eluent: 0.1% ammonia water solution) to obtain 700 mg of tert-butyl (tert-butoxycarbonyl)(4-chloro-7-((4-oxo-2-thio-2,3,4,5-tetrahydro-1H-pyrrolo[3,2-d]pyrimi din-1-yl)methyl)-2,3-dihydro-1H-inden-1-yl (yield: 76%). ¹H NMR (400 MHz, DMSO) δ 12.51 (s, 1H), 12.38 (d, *J*=8.0 Hz, 1H), 7.32 (t, *J*=2.8 Hz, 1H), 7.19 (dd, *J*=13.6, 8.0 Hz, 1H), 6.57 (t, *J*=7.2 Hz, 1H), 6.11 (dd, *J*=9.6, 4.4 Hz, 1H), 5.89 (dd, *J*=9.6, 7.2 Hz, 1H), 5.78 (d, *J*=16.4 Hz, 1H), 5.29 (d, *J=16.4* Hz, 1H), 3.11 (ddd, *J*=16.0, 10.0, 5.6 Hz, 1H), 2.97-2.76 (m, 1H), 2.63 (ddd, *J*=20.0, 12.4, 7.4 Hz, 1H), 2.17 (td, *J*=9.9, 5.1 Hz, 1H), 1.33 (s, 18H).

### Step L: synthesis of 1-((3-amino-7-chloro-2,3-dihydro-1H-inden-4-yl)methyl)-2-thio-1,2,3,5-tetrahydro-4H-pyrrolo [3,2-d] pyrimidin-4-one

Tert-butyl (tert-butoxycarbonyl)(4-chloro-7-((4-oxo-2-thio-2,3,4,5-tetrahydro-1H-pyrrolo[3,2-d]pyrimi din-1-yl)methyl)-2,3-dihydro-1H-inden-1-yl (80 mg, 0.14 mmol) was dissolved in ethyl acetate (2 mL), an ethyl acetate hydrochloride solution (2 mL) was added to a reaction solution, and the reaction solution was stirred at a room temperature for 1 hour.

A solvent in the reaction solution was subjected to spin drying. Then a sodium bicarbonate aqueous solution was used to adjust a pH of the solution to 8, ethyl acetate (15 mL×2) was used for extraction, and organic phases were combined, dried with anhydrous sodium sulfate, filtered, and subjected to spin drying. The resulting residue was purified using a reversed-phase column (an eluent: 0.1% ammonia water solution) to obtain 30 mg of 1-((3-amino-7-chloro-2,3-dihydro-1H-inden-4-yl)methyl)-2-thio-1,2,3,5-tetrahydro-4H-pyrr olo[3,2-d]pyrimidin-4-one (yield: 60%). LC-MS: [M+H]⁺=347.2. ¹H NMR (400 MHz, DMSO) δ 7.19 (dd, *J*=14.1, 2.6 Hz, 1H), 7.10 (dd, *J*=8.2, 3.9 Hz, 1H), 6.56 (dd, *J*=23.0, 16.3 Hz, 1H), 6.07 (d, *J*=2.7 Hz, 1H), 5.99-5.89 (m, 1H), 5.79 (d, *J*=16.4 Hz, 1H), 4.60 (dd, *J*=7.1, 3.0 Hz, 1H), 3.13-2.96 (m, 1H), 2.84-2.66 (m, 1H), 2.37 (dt, *J*=16.1, 7.5 Hz, 1H), 1.84 (ddd, *J*=12.4, 8.3, 4.0 Hz, 1H).

### Step M: synthesis of compounds M-1 and M-2

1-((3-amino-7-chloro-2,3-dihydro-1H-inden-4-yl)methyl)-2-thio-1,2,3,5-tetrahydro-4H -pyrrolo[3,2-d]pyrimidin-4-one (120 mg, 0.34 mmol), di-tert-butyl dicarbonate (83 mg, 0.38 mmol), and triethylamine (103 mg, 1.02 mmol) were dissolved in dichloromethane (2 mL), and a mixture reacted at a room temperature for 2 hours.

10 mL of water was added to a reaction solution, dichloromethane (10 mL×2) was used for extraction, and organic phases were combined, washed with a NaHCO₃ aqueous solution, dried with anhydrous sodium sulfate, filtered, and subjected to spin drying. The resulting residue was purified using a reversed-phase column (an eluent: 0.1% ammonia water solution) to obtain 96 mg of tert-butyl 1-((3-(tert-butoxycarbonyl)amino)-7-chloro-2,3-dihydro-1H-inden-4-yl)methyl)-4-oxo-2-thi o-1,2,3,4-tetrahydro-5H-pyrrole[3,2-d]pyrimidine-5-carboxylate. The white solid was fed to SFC for separation (DAICELCHIRALCEL^{®}IC column (250*25 mm 10 um), Supercritical CO2 and MEOH (+0.1% 7.0 mol/l Ammonia in MEOH) were used as mobile phases for chiral separation preparation, so as to obtain an isomer M-1(RT=2.4 min,20 mg, yield=21%, ee>99%) and M-2(RT=3.4 min, 15 mg, yield=16%, ee>99%).

### Step N:

M-1 (20 mg, 0.036 mmol) was dissolved in ethyl acetate (2 mL), an ethyl acetate hydrochloride solution (2 mL) was added to a reaction solution, and the reaction solution was stirred at a room temperature for 1 hour. A solvent in the reaction solution was subjected to spin drying. The reaction solution was subjected to spin drying. Then a sodium bicarbonate aqueous solution was used to adjust a pH of the solution to 8, ethyl acetate (15 mL×2) was used for extraction, and organic phases were combined, dried with anhydrous sodium sulfate, filtered, and subjected to spin drying. The resulting residue was purified using a reversed-phase column (an eluent: 0.1% ammonia water solution), and after freeze-drying, 10.6 mg of compound 1A was obtained (yield 84%). LC-MS: [M+H]⁺=347.2

M-2 (15 mg, 0.027 mmol) was dissolved in ethyl acetate (2 mL), an ethyl acetate hydrochloride solution (2 mL) was added to a reaction solution, and the reaction solution was stirred at a room temperature for 1 hour. A solvent in the reaction solution was subjected to spin drying. Then a sodium bicarbonate aqueous solution was used to adjust a pH of the solution to 8, ethyl acetate (15 mL×2) was used for extraction, and organic phases were combined, dried with anhydrous sodium sulfate, filtered, and subjected to spin drying. The resulting residue was purified using a reversed-phase column (an eluent: 0.1% ammonia water solution), and after freeze-drying, 8.5 mg of compound **1B** was obtained (yield 89%). LC-MS: [M+H]⁺=347.2.

### Example 2

### 1-((3-amino-7-chloro-2,3-dihydrobenzofuran-4-yl)methyl)-2-thio-1,2,3,5-tetrahydro-4H -pyrrolo[3,2-d]pyrimidin-4-one

### Synthetic route:

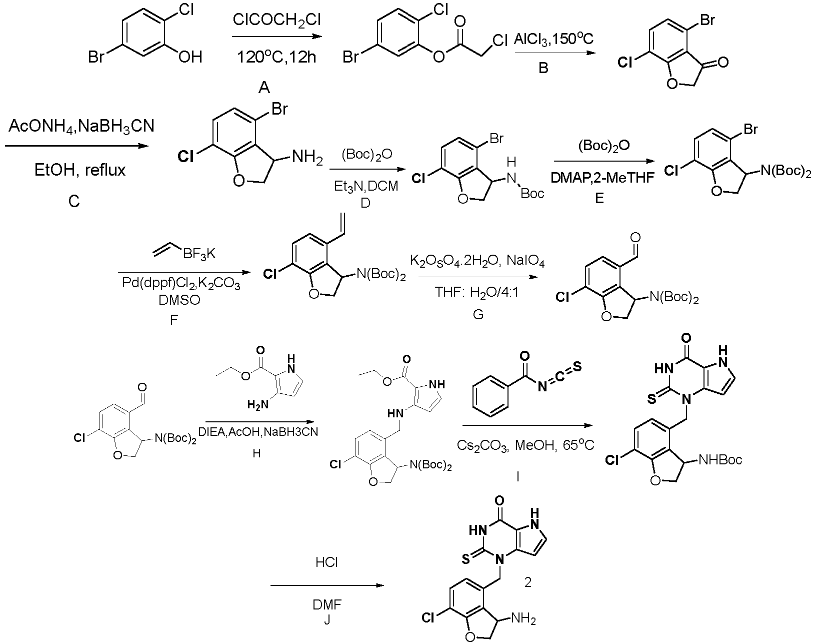

### Step A: 5-bromo-2-chlorophenyl 2-chloroacetate

5-bromo-2-chlorophenol (12 g, 0.058 mol) and chloroacetyl chloride (19.6 g, 0.774 mol) were added to a three-necked flask and heated to reflux for 16 hours.

After the reaction was completed, a reaction solution was subjected to spin drying, and a crude product was purified using a silica gel column (petroleum ether:ethyl acetate=100:1 to 10:1) to obtain 16 g (0.056 mol) of a white solid product 5-bromo-2-chlorophenyl 2-chloroacetate (yield: 97.1%).¹H NMR (400 MHz, DMSO) δ 7.73 (d, J=1.6 Hz, 1H), 7.61-7.57 (m, 2H), 4.78 (s, 2H).

### Step B: synthesis of 4-bromo-7-chlorophenofuran-3(2H)-one

5-bromo-2-chlorophenyl 2-chloroacetate (16 g, 0.056 mol) was added to a three-necked flask, replaced with nitrogen, and heated to 150°C, AlCl₃ (22.54 g, 0.168 mol) was added, and a mixture reacted at 150°C for 0.5 hour.

After the reaction was completed, ice water (50 mL) was added for quenching, ethyl acetate (50 mL×3) was used for extraction, and the mixture was washed with a saturated sodium chloride solution (30 mL×3), dried with anhydrous sodium sulfate, filtered, and subjected to spin drying to obtain a crude product. The crude product was purified using a silica gel column (petroleum ether:ethyl acetate=100:1 to 10:1) to obtain 3.2 g (0.013 mol) of 4-bromo-7-chlorophenofuran-3(2H)-one (yield: 23.1%). ¹H NMR (400 MHz, DMSO) δ 7.74 (d, *J*=8.3 Hz, 1H), 7.34 (d, *J*=8.3 Hz, 1H), 4.95 (s, 2H).

### Step C: synthesis of 4-bromo-7-chloro-2,3-dihydrobenzofuran-3-amine

4-bromo-7-chlorophenofuran-3(2H)-one (3.2 g, 12.95 mmol) in was dissolved in an EtOH solution (20 mL) at a room temperature, then ammonium acetate (9.98 g, 129.5 mmol) was added, and a mixture was heated to 100°C for a reaction for 12 hours.

After the reaction was completed, ice water (20 mL) was added for quenching, then ethanol in a reaction solution was subjected to spin drying, a saturated sodium bicarbonate solution was added to adjust a pH to 4-5, ethyl acetate (20 mL×3) was used for extraction, and the reaction solution was washed with a saturated sodium chloride solution (30 mL×3), dried with anhydrous sodium sulfate, filtered, and subjected to spin drying to obtain a crude product. The crude product was purified by C18 reverse phase column chromatography (NH4CO3/CAN system) to obtain 1.5 g (6.04 mmol) of

4-bromo-7-chloro-2,3-dihydrobenzofuran-3-amine (yield: 46.9%) LC-MS: [M+H]⁺=248.50. ¹H NMR (400 MHz, DMSO) δ 7.30 (d, *J*=8.5 Hz, 1H), 7.10 (d, *J*=8.5 Hz, 1H), 5.91 (d, *J*=7.2 Hz, 1H), 5.25 (td, *J*=6.9, 2.0 Hz, 1H), 4.63 (dd, *J*=10.4, 6.5 Hz, 1H), 4.42 (dd, *J*=10.4, 2.1 Hz, 1H).

### Step D: synthesis of tert-butyl(4-bromo-7-chloro-2,3-dihydrobenzofuran-3-yl)carbamate

At a room temperature, 4-bromo-7-chloro-2,3-dihydrobenzofuran-3-amine (1.5 g, 6.03 mmol) was dissolved in dichloromethane (20 mL), (Boc)2O (2.63 g, 12.07 mmol) and TEA (2.5 mL, 18.09 mmol) were added, and a mixture was stirred at a room temperature for 12 hours.

At the end of the reaction, saturated sodium bicarbonate (20 mL) was added to quench the reaction, DCM extraction (10 mL×3) was performed, organic phases were combined, dried with anhydrous sodium sulfate, filtered, and concentrated to dryness, and the resulting residue was purified by silica gel column chromatography (NH₄CO₃/CAN system) to obtain 1.2 g (3.44 mmol) tert-butyl(4-bromo-7-chloro-2,3-dihydrobenzofuran-3-yl)carbamate (yield 57.1%). LC-MS: [M+H]⁺=348.12. ¹H NMR (400 MHz, DMSO) δ 7.60 (d, *J*=8.6 Hz, 1H), 7.27 (d, *J*=8.5 Hz, 1H), 7.08 (d, *J*=8.5 Hz, 1H), 5.38-5.30 (m, 1H), 4.78 (t, *J*=9.2 Hz, 1H), 4.32 (dd, *J*=9.5, 4.1 Hz, 1H), 1.39 (s, 9H).

### Step E: synthesis of tert-butyl(4-bromo-7-chloro-2,3-dihydrobenzofuran-3-yl)(tert-butoxycarbonyl)carbam ate

At a room temperature,
tert-butyl(4-bromo-7-chloro-2,3-dihydrobenzofuran-3-yl)carbamate (1.2 g, 3.45 mmol) was dissolved in dichloromethane (20 mL), (Boc)2O (1.5 g, 6.9 mmol) and DMAP (0.84 g, 6.9 mmol) were added, and a mixture was stirred at a room temperature for 12 hours.

At the end of the reaction, saturated sodium bicarbonate (20 mL) was added to quench the reaction, DCM extraction (10 mL×3) was performed, organic phases were combined, dried with anhydrous sodium sulfate, filtered, and concentrated to dryness, and the resulting residue was purified by C18 reverse phase column chromatography (NH4CO3/CAN system) to obtain 1.2 g (2.67 mmol) tert-butyl(4-bromo-7-chloro-2,3-dihydrobenzofuran-3-yl)(tert-butoxycarbonyl)carbamate (yield 80%). LC-MS: [M+H]⁺=448.74. ¹H NMR (400 MHz, DMSO) δ 7.31-7.25 (m, 1H), 7.07 (d, *J*=8.5 Hz, 1H), 6.19 (dd, *J*=10.2, 4.8 Hz, 1H), 4.91-4.84 (m, 1H), 4.58 (dd, *J*=9.8, 4.9 Hz, 1H), 1.43-1.20 (m, 18H).

### Step F: synthesis of (tert-butyl(tert-butoxycarbonyl)(7-chloro-4-vinyl-2,3-dihydrobenzofuran-3-yl)carbama te

(Tert-butyl(tert-butoxycarbonyl)(7-chloro-4-vinyl-2,3-dihydrobenzofuran-3-yl)carbama te (1.2 g, 2.68 mmol), potassium vinyltrifluoroborate (0.54 g, 4.01 mmol), 1,1'-bis(diphenylphosphine)ferrocene palladium chloride (0.22 g, 0.3 mmol), and potassium carbonate (1.11 g, 8.04 mmol) were dissolved in dimethyl sulfoxide (15 mL), and a mixture reacted at 100°C under nitrogen protection for 12 hours.

At the end of the reaction, the mixture was cooled to a room temperature, a reaction solution was filtered through diatomaceous earth, filtrate was collected, then 50 mL of water was added, ethyl acetate (50 mL×3) was used for extraction, and organic phases were combined, washed with a NaCl aqueous solution, dried with anhydrous sodium sulfate, filtered, and subjected to spin drying. The resulting residue was purified by silica gel column chromatography (an eluent: petroleum ether/ethyl acetate=10/1) to obtain 0.552 g (1.32 mmol) of (tert-butyl(tert-butoxycarbonyl)(7-chloro-4-vinyl-2,3-dihydrobenzofuran-3-yl)carbamate (yield: 55%). LC-MS: [M+H]⁺=395.88. ¹H NMR (400 MHz, DMSO) δ 7.30 (d, *J*=8.4 Hz, 1H), 7.13 (d, *J*=8.4 Hz, 1H), 6.62 (dd, *J*=17.6, 11.1 Hz, 1H), 6.24 (dd, *J*=9.9, 4.3 Hz, 1H), 5.83 (d, *J*=17.5 Hz, 1H), 5.35 (d, *J*=11.2 Hz, 1H), 4.80 (t, *J*=9.9 Hz, 1H), 4.57 (dd, *J*=9.9, 4.4 Hz, 1H), 1.29 (s, 18H).

### Step G: synthesis of tert-butyl (tert-butoxycarbonyl)(7-chloro-4-formyl-2,3-dihydrobenzofuran-3-yl)carbamate

(Tert-butyl(tert-butoxycarbonyl)(7-chloro-4-vinyl-2,3-dihydrobenzofuran-3-yl)carbama te (522 mg, 1.39 mmol) was dissolved in a mixed solution of tetrahydrofuran (4 mL) and water (1 mL), potassium osmate (51.49 mg, 0.14 mmol) was added to a reaction solution, the reaction solution was stirred at a room temperature for 30 minutes, then sodium periodate (1.2 g, 5.56 mmol) was added to the reaction solution, and the reaction solution reacted at a room temperature for 2 hours.

At the end of the reaction, 20 mL of water was added to the reaction solution, ethyl acetate (20 mL×4) was used for extraction, and organic phases were combined, dried with anhydrous sodium sulfate, filtered, and subjected to spin drying. The resulting residue was purified by silica gel column chromatography (an eluent: petroleum ether/ethyl acetate=10/1) to obtain 315 mg (0.79 mmol) of tert-butyl (tert-butoxycarbonyl)(7-chloro-4-formyl-2,3-dihydrobenzofuran-3-yl)carbamate (yield: 56.9%). LC-MS: [M+H]⁺=397.85. ¹H NMR (400 MHz, DMSO) δ 9.97 (s, 1H), 7.62 (d, *J*=8.2 Hz, 1H), 7.46 (d, *J*=8.2 Hz, 1H), 6.45 (dd, *J*=10.1, 4.7 Hz, 1H), 4.92 (t, *J*=10.1 Hz, 1H), 4.61 (dd, *J*=9.9, 4.7 Hz, 1H), 1.31 (s, 18H).

### Step H: synthesis of ethyl 3-(((3-(bis(tert-butoxycarbonyl)amino)-7-chloro-2,3-dihydrobenzofuran-4-yl)methyl)a mino)-1H-pyrrole-2-carboxylate

3-amino-2-ethoxycarbonylpyrrole hydrochloride (181.2 mg, 0.951 mmol) was dissolved in 10 mL of anhydrous ethanol, then N,N-diisopropylethylamine (122.83 mg, 0.951 mmol) and glacial acetic acid (57.1 mg, 0.951 mmol) were added to a mixture, the mixture was stirred at a room temperature for 10 minutes, then tert-butyl (tert-butoxycarbonyl)(7-chloro-4-formyl-2,3-dihydrobenzofuran-3-yl)carbamate (315 mg, 0.792 mmol) was added to the mixture, and stirred at a room temperature for 2 hours, then sodium cyanoborohydride (59.8 mg, 0.95 mmol) was added to the mixture, and the mixture reacted at a room temperature for 4 hours.

At the end of the reaction, the anhydrous ethanol in the reaction solution was subjected to spin drying. 10 mL of water and ethyl acetate (20 mL×2) were used for extraction, and organic phases were combined, dried with anhydrous sodium sulfate, filtered, and subjected to spin drying. The resulting residue was purified using a reversed-phase column (an eluent: 0.1% ammonia water solution) to obtain 221 mg (0.41 mmol) of ethyl 3-(((3-(bis(tert-butoxycarbonyl)amino)-7-chloro-2,3-dihydrobenzofuran-4-yl)methyl)amino) -1H-pyrrole-2-carboxylate (yield: 52.0%). LC-MS: [M+H]⁺=536.02. ¹H NMR (400 MHz, DMSO) δ 10.77 (s, 1H), 7.31-7.18 (m, 1H), 6.81 (d, *J*=7.8 Hz, 1H), 6.69 (s, 1H), 6.24 (d, *J*=9.8 Hz, 1H), 5.79 (s, 1H), 5.46 (s, 1H), 4.82 (t, *J*=8.7 Hz, 1H), 4.61 (d, *J*-2.9 Hz, 1H), 4.28-4.08 (m, 4H), 1.27 (d, J=12.0 Hz, 21H).

### Step I: synthesis of tert-butyl(7-chloro-4-((4-oxo-2-thio-2,3,4,5-tetrahydro-1H-pyrrolo[3,2-d]pyrimidin-1-y 1)methyl)-2,3-dihydrobenzofuran-3-yl)carbamate

Ethyl3-(((3-(bis(tert-butoxycarbonyl)amino)-7-chloro-2,3-dihydrobenzofuran-4-yl)met hyl)amino)-1H-pyrrole-2-carboxylate (221 mg, 0.41 mmol) and benzoyl isothiocyanate (80.75 mg, 0.49 mmol) ware dissolved in methanol (5 mL), a mixture was stirred at a room temperature for 3 hours, then cesium carbonate (537.4 mg, 1.64 mmol) was added, and a reaction solution reacted at 65°C for 2 hours.

At the end of the reaction, the anhydrous methanol in the reaction solution was subjected to spin drying. 20 mL of water and ethyl acetate (20 mL×2) were used for extraction, and organic phases were combined, dried with anhydrous sodium sulfate, filtered, and subjected to spin drying. The resulting residue was purified using a reversed-phase column (an eluent: 0.1% ammonia water solution) to obtain 65 mg (0.14 mmol) of tert-butyl(7-chloro-4-((4-oxo-2-thio-2,3,4, 5-tetrahydro-1H-pyrrolo[3,2-d]pyrimidin-1-yl)met hyl)-2,3-dihydrobenzofuran-3-yl)carbamate (yield: 35.1%). LC-MS: [M+H]+=448.92.¹H NMR (400 MHz, DMSO) δ 12.50 (s, 1H), 12.39 (s, 1H), 7.84 (d, *J*=8.7 Hz, 1H), 7.32 (s, 1H), 7.16 (d, *J*=8.3 Hz, 1H), 6.28 (d, *J*=8.3 Hz, 1H), 6.00 (s, 1H), 5.72 (d, J=16.7 Hz, 1H), 5.67-5.60 (m, 1H), 5.48 (d, *J*=16.4 Hz, 1H), 4.81 (t, *J*=9.3 Hz, 1H), 4.40 (d, J=5.5 Hz, 1H), 1.39 (s, 9H).

### Step J: synthesis of 1-((3-amino-7-chloro-2,3-dihydrobenzofuran-4-yl)methyl)-2-thio-1,2,3,5-tetrahydro-4H -pyrrolo[3,2-d]pyrimidin-4-one

Tert-butyl(7-chloro-4-((4-oxo-2-thio-2,3,4,5-tetrahydro-1H-pyrrolo[3,2-d]pyrimidin-1-yl)methyl)-2,3-dihydrobenzofuran-3-yl)carbamate (65 mg, 0.15 mmol) was dissolved in DMF (2 mL), 1,4-dioxane hydrochloride solution (5 mL) was added to a reaction solution, and the reaction solution was stirred at a room temperature for 1 hour. 10 mL of water was added to a reaction solution, dichloromethane (10 mL×2) was used for extraction, and organic phases were combined, washed with a NaHCO₃ aqueous solution, dried with anhydrous sodium sulfate, filtered, and subjected to spin drying. The resulting residue was purified using a reversed-phase column (an eluent: 0.1% ammonia water solution) to obtain 24.01 mg of 1-((3-amino-7-chloro-2,3-dihydrobenzofuran-4-yl)methyl)-2-thio-1,2,3,5-tetrahydro-4H-pyr rolo[3,2-d]pyrimidin-4-one (yield: 47.5%). LC-MS: [M+H]⁺=348.81. ¹HNMR (400 MHz, DMSO) δ 7.30 (d, *J*=2.8 Hz, 1H), 7.12 (d, *J*=8.3 Hz, 1H), 6.31 (d, *J*=8.3 Hz, 1H), 6.16 (d, *J*=2.8 Hz, 1H), 5.88 (d, *J*=16.6 Hz, 1H), 5.76 (d, *J*=16.6 Hz, 1H), 4.92-4.83 (m, 1H), 4.71 (t, *J*=8.7 Hz, 1H), 4.30 (dd, *J*=9.4, 4.1 Hz, 1H).

### Example 3

### 1-(3-amino-2,3-dihydrobenzofuran-4-yl)methyl)-2-thio-1,2,3,5-tetrahydro-4H-pyrrolo[ 3,2-d]pyrimidin-4-one

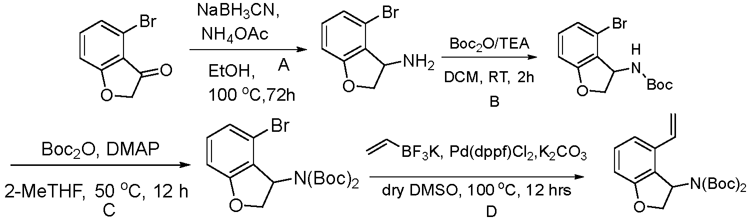

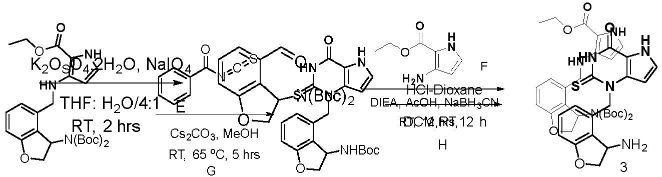

### Step A: synthesis of 4-bromo-2,3-dihydrobenzofuran-3-amine

At a room temperature, 4-bromobenzofuran-3(2H) (2.4 g, 11.26 mmol), ammonium acetate (8.7 g, 112.6 mmol), and sodium cyanoborohydride (3.54 g, 56.3 mmol) were dissolved in ethanol (30 mL), and a reaction was performed in a sealed reaction tube at 100°C for 72 hours.

At the end of the reaction, a reaction solution was subjected to spin drying on a water pump, the reaction solution (30 mL×3) was extracted with ethyl acetate and water, and organic phases were combined, dried with anhydrous sodium sulfate, filtered, and concentrated to dryness to obtain 2.2 g of oily-liquid crude product 4-bromo-2,3-dihydrobenzofuran-3-amine (yield 91.28%). LC-MS: [M+H]+=214.06.

### Step B: synthesis of tert-butyl(4-bromo-2,3-dihydrobenzofuran-3-yl)carbamate

At a room temperature, 4-bromo-2,3-dihydrobenzofuran-3-amine (2.2 g, 10.33 mmol), (Boc)₂O (4.74 mL, 20.66 mmol), and triethylamine (2.9 mL, 20.66 mmol) were dissolved in DCM (30 mL), and a mixture was stirred at a room temperature for 2 hours.

At the end of the reaction, water (30 mL) was added, dichloromethane (30 mL×3) was used for extraction, organic phases were combined, dried with anhydrous sodium sulfate, filtered, and concentrated to dryness, and the resulting residue was purified by silica gel column chromatography (an eluent: petroleum ether:ethyl acetate=20:1) to obtain 1.6 g of oily-liquid product tert-butyl(4-bromo-2,3-dihydrobenzofuran-3-yl)carbamate (yield 53.12%). LC-MS: [M+H]+=314.18. ¹H NMR (400 MHz, DMSO) δ 7.54 (d, J=8.5 Hz, 1H), 7.15 (t, J=7.9 Hz, 1H), 7.06 (d, J=7.9 Hz, 1H),6.84 (t, J=9.5 Hz, 1H), 5.29 (dt, J=12.3, 6.3 Hz, 1H), 4.67 (t, J=8.2 Hz, 1H), 4.23 (dd, J=9.5, 4.0 Hz, 1H), 1.44 (d, J=28.9 Hz, 9H).

### Step C: synthesis of tert-butyl(4-bromo-2,3-dihydrobenzofuran-3-yl)(tert-butoxycarbonyl)carbamate

At 50°C, tert-butyl(4-bromo-2,3-dihydrobenzofuran-3-yl)carbamate (1.6 g, 5.11 mmol), (Boc)₂O (11.68 mL, 51.2 mmol), and DMAP (1.25 mL, 10.24 mmol) were dissolved in 2-methyltetrahydrofuran (15 mL), and a mixture was stirred at a 50°C for 12 hours.

At the end of the reaction, water (30 mL) was added, ethyl acetate (30 mL×3) was used for extraction, organic phases were combined, dried with anhydrous sodium sulfate, filtered, and concentrated to dryness, and the resulting residue was purified by silica gel column chromatography (an eluent: petroleum ether:ethyl acetate=20:1) to obtain 2 g of oily-liquid product tert-butyl(4-bromo-2,3-dihydrobenzofuran-3-yl)(tert-butoxycarbonyl)carbamate (yield 94.78%). LC-MS: [M+H]+=414.30. ¹H NMR (400 MHz, DMSO) δ 7.27-7.11 (m, 1H), 7.06 (t, J=12.2 Hz, 1H), 6.83 (t, J=10.9 Hz, 1H), 6.10 (dd, J=10.1, 4.8 Hz, 1H), 4.77 (t, J=10.0 Hz, 1H), 4.57-4.44 (m, 1H), 1.61-1.19 (m, 18H).

### Step D: synthesis of tert-butyl(4-vinyl-2,3-dihydrobenzofuran-3-yl)carbamate

Tert-butyl(4-bromo-2,3-dihydrobenzofuran-3-yl)(tert-butoxycarbonyl)carbamate (2 g, 4.84 mmol), potassium trifluoroborate (973 mg, 7.26 mmol), potassium carbonate (3.23 g, 23.43 mmol), and [1,1'-bis(diphenylphosphino)ferrocene]dichloropalladium(II) (351 mg, 0.484 mmol) were dissolved in 30 mL of 1,4-dioxane and 3 mL of water, and a reaction was performed at 100°C for 12 hours.

At the end of the reaction, water (30 mL) was added, ethyl acetate (30 mL×3) was used for extraction, organic phases were combined, dried with anhydrous sodium sulfate, filtered, and concentrated to dryness, and the resulting residue was purified by silica gel column chromatography (an eluent: petroleum ether:ethyl acetate=20:1) to obtain 1.16 g of oily-liquid product tert-butyl(4-vinyl-2,3-dihydrobenzofuran-3-yl)carbamate (yield 66.36%). LC-MS: [M+H]⁺=361.44. ¹H NMR (400 MHz, DMSO) δ 7.28-7.17 (m, 1H), 7.10 (d, J=7.7 Hz, 1H), 6.79-6.62 (m, 2H), 6.14 (dd, J=9.9, 4.2 Hz, 1H), 5.88-5.68 (m, 1H), 5.31 (dd, J=11.1, 0.8 Hz, 1H), 4.69 (t, J=9.9 Hz, 1H), 4.48 (dd, J=9.8, 4.3 Hz, 1H), 1.28 (s, 18H).

### Step E: synthesis of tert-butyl(4-formyl-2,3-dihydrobenzofuran-3-yl)carbamate

At a room temperature, tert-butyl(4-vinyl-2,3-dihydrobenzofuran-3-yl)carbamate (1.16 g, 3.21 mmol) was dissolved in a mixed solution of tetrahydrofuran (12 mL) and water (3 mL), potassium osmate (59 mg, 0.16 mmol) was added to a reaction solution, the reaction solution was stirred at a room temperature for 30 minutes, then sodium periodate (2.08 g, 9.63 mmol) was added to the reaction solution, and the reaction solution reacted at a room temperature for 1.5 hours.

At the end of the reaction, water (30 mL) was added, ethyl acetate (30 mL×3) was used for extraction, organic phases were combined, dried with anhydrous sodium sulfate, filtered, and concentrated to dryness, and the resulting residue was purified by silica gel column chromatography (an eluent: petroleum ether:ethyl acetate=20:1) to obtain 430 mg of oily-liquid product tert-butyl(4-formyl-2,3-dihydrobenzofuran-3-yl)carbamate (yield 36.87%). LC-MS: [M+H]+=363.41. ¹H NMR (400 MHz, DMSO) δ 10.01 (s, 1H), 7.57-7.38 (m, 2H), 7.16 (d, *J*=7.8 Hz, 1H), 6.34 (dt, *J*=61.4, 30.8 Hz, 1H), 4.91-4.71 (m, 1H), 4.52 (dd, *J*=9.8, 4.6 Hz, 1H), 1.33 (d, *J*=23.6 Hz, 18H).

### Step F: synthesis of ethyl 3-((3-(bis(tert-butoxycarbonyl)amino)-2,3-dihydrobenzofuran-4-yl)methyl)amino)-1H-pyrrole-2-carboxylate

At a room temperature, 3-amino-2-ethoxycarbonylpyrrole hydrochloride (271 mg, 1.42 mmol) and DIEA (0.22 mL, 1.42 mmol) were added to ethanol (20 mL) and allowed to react for 5 minutes, glacial acetic acid (0.11 mL, 1.42 mmol) was added, and a mixture reacted at a room temperature for 30 minutes; tert-butyl(4-formyl-2,3-dihydrobenzofuran-3-yl)carbamate (430 mg, 1.18 mmol) was added, and a mixture reacted at a room temperature for 1 hour; and sodium cyanoborohydride (219 mg, 2.48 mmol) was added, and a mixture reacted at a room temperature for 12 hours.

At the end of the reaction, ethanol in a reaction solution was subjected to spin drying, water (20 mL) was added, ethyl acetate (20 mL×3) was used for extraction, organic phases were combined, dried with anhydrous sodium sulfate, filtered, and concentrated to dryness, and the resulting residue was purified by reverse phase column chromatography (an eluent: 0.1% ammonium bicarbonate aqueous solution:acetonitrile=0-50%) to obtain 450 mg of ethyl3-((3-(bis(tert-butoxycarbonyl)amino)-2,3-dihydrobenzofuran-4-yl)methyl)amino)-1H-pyrrole-2-carboxylate (yield 75.88%). LC-MS: [M+H]⁺=501.58. ¹H NMR (400 MHz, DMSO) δ 10.76 (s, 1H), 7.17 (t, *J*=7.8 Hz, 1H), 6.89-6.59 (m, 3H), 6.14 (dd, *J*=9.8, 4.1 Hz, 1H), 5.75 (s, 1H), 5.51 (t, *J* -2.5 Hz, 1H), 4.69 (t, *J*=9.9 Hz, 1H), 4.51 (dd, *J*=9.9, 4.2 Hz, 1H), 4.33-4.06 (m, 4H), 1.27 (d, *J*=3.7 Hz, 18H), 1.24 (d, *J*=6.3 Hz, 3H).

### Step G: synthesis of tert-butyl(4-(4-oxo-2-thio-2,3,4,5-tetrahydro-1H-pyrrolo[3,2-d]pyrimidin-1-yl)methyl)-2,3-dihydrobenzofuran-3-yl)carbamate

At a room temperature, ethyl
3-((3-(bis(tert-butoxycarbonyl)amino)-2,3-dihydrobenzofuran-4-yl)methyl)amino)-1H-pyrro le-2-carboxylate (450 mg, 0.9 mmol) and benzoyl isothiocyanate (0.18 mL, 1.08 mmol) ware added to 30 mL of anhydrous methanol, a mixture reacted at a room temperature for 2 hours, then cesium carbonate (1.18 g, 3.6 mmol) was added, and a mixture reacted at 65°C for 2 hours.

At the end of the reaction, methanol in a reaction solution was subjected to spin drying, water (20 mL) was added, ethyl acetate (30 mL×3) was used for extraction, organic phases were combined, dried with anhydrous sodium sulfate, filtered, and concentrated to dryness, and the resulting residue was purified by reverse phase column chromatography (an eluent: 0.1% ammonium bicarbonate aqueous solution:acetonitrile=0-50%) to obtain 300 mg of tert-butyl(4-(4-oxo-2-thio-2,3,4,5-tetrahydro-1H-pyrrolo[3,2-d]pyrimidin-1-yl)methyl)-2,3-d ihydrobenzofuran-3-yl)carbamate (yield 65.07%). LC-MS: [M+H]⁺=414.48. ¹H NMR (400 MHz, DMSO) δ 7.75 (d, *J*-8.3 Hz, 1H), 7.16-6.92 (m, 2H), 6.66 (d, *J*=7.9 Hz, 1H), 6.29 (d, *J*=7.7 Hz, 1H), 5.92-5.64 (m, 2H), 5.56 (d, *J*=15.4 Hz, 2H), 4.69 (t, *J*=9.2 Hz, 1H), 4.35-4.20 (m, 1H), 1.39 (s, 9H).

### Step H: synthesis of 1-(3-amino-2,3-dihydrobenzofuran-4-yl)methyl)-2-thio-1,2,3,5-tetrahydro-4H-pyrrolo[ 3,2-d]pyrimidin-4-one

At a room temperature,
tert-butyl(4-(4-oxo-2-thio-2,3,4,5-tetrahydro-1H-pyrrolo[3,2-d]pyrimidin-1-yl)methyl)-2,3-d ihydrobenzofuran-3-yl)carbamate (300 mg, 0.58 mmol) was dissolved in DCF (20 mL), 1,4-dioxane hydrochloride (4.38 mL, 17.5 mmol) was added, and a mixture reacted at a room temperature for 12 hours.

At the end of the reaction, a mixture was beaten with dichloromethane for 1 hour and filtered to obtain 209 mg of solid 1-(3-amino-2,3-dihydrobenzofuran-4-yl)methyl)-2-thio-1,2,3,5-tetrahydro-4H-pyrrolo[3,2-d ]pyrimidin-4-one hydrochloride. LC-MS: [M+H]+=314.36. ¹H NMR (400 MHz, DMSO) δ 12.45 (d, *J*=46.4 Hz, 2H), 8.69 (s, 3H), 7.27 (dt, *J*=15.9, 5.4 Hz, 2H), 6.86 (d, *J*=8.0 Hz, 1H), 6.38 (d, *J*=7.7 Hz, 1H), 6.12 (dd, *J*=12.9, 10.3 Hz, 2H), 5.65 (d, *J*=16.7 Hz, 1H), 5.35 (s, 1H), 4.78-4.60 (m, 2H).

### Example 4

Referring to a preparation method of Example 1, compound 4 of the following formula was prepared. 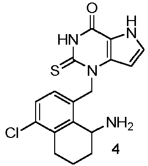 LC-MS: [M+H]+=361.1.

### Example 5

### 1-((4-amino-8-chlorochroman-5-yl)methyl)-2-thio-1,2,3,5-tetrahydro-4H-pyrrolo[3,2-d] pyrimidin-4-one

### Synthetic route:

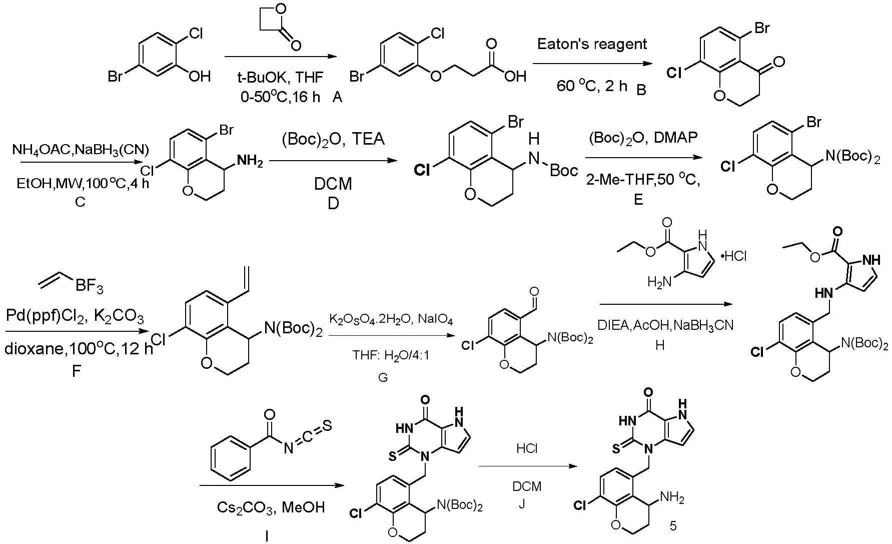

### Step A: 3-(5-bromo-2-chlorophenoxy)propanoic acid

At a room temperature, 5-bromo-2-chlorophenol (3 g, 0.014 mol) was dissolved in THF (40 mL), a mixture was cooled to 0°C, t-BuOK (1.79 g, 0.16 mol) was added, a reaction was performed at 0°C for 0.5 hour, amyleneoxide-2-one (1.15 g, 0.016 mol) was added, and a mixture was raised to a room temperature for a reaction for 16 hours.

At the end of the reaction, ice water (10 mL) was added to quench the reaction, ethyl acetate (30 mL×3) was used for extraction, organic phases were combined, dried with anhydrous sodium sulfate, filtered, and concentrated to dryness, so as to obtain 4.7 g (147 mmol) of crude 3-(5-bromo-2-chlorophenoxy)propanoic acid as a white solid product.

### Step B: 5-bromo-8-chlorochroman--4-one

At a room temperature, 3-(5-bromo-2-chlorophenoxy)propanoic acid (1.5 g, 5.4 mmol) was dissolved in an Eaton's reagent (7 mL), and a mixture was heated and stirred at 60°C for 2 hours.

At the end of the reaction, ice water (10 mL) was added to quench the reaction, ethyl acetate (30 mL×3) was used for extraction, organic phases were combined, dried with anhydrous sodium sulfate, filtered, and concentrated to dryness, and a crude product was purified by a silica gel column (PE:EA=100:1 to 10:1) to obtain 1 g (1.47 mmol) of 5-bromo-8-chlorochroman--4-one (yield 70.8%). ¹H NMR (400 MHz, DMSO) δ 7.59 (d, *J*=8.5 Hz, 1H), 7.32 (d, *J*=8.5 Hz, 1H), 4.68 (t, *J*=6.5 Hz, 2H), 2.93-2.83 (m, 2H).

### Step C: synthesis of 5-bromo-8-chlorochroman--4-amine

At a room temperature, 5-bromo-8-chlorochroman-4-one (1 g, 3.82 mmol) was dissolved in EtOH (10 mL), AcONH₄ (2.95 g, 38.2 mmol) and NaBH₃CN (1.2 g, 19.1 mmol) were added, and a mixture was stirred at 100°C for 16 hours.

At the end of the reaction, ethanol was removed by spin drying, water (20 mL) and saturated NaHCO₃ (10 mL) were added, ethyl acetate (50 mL×3) was used for extraction, organic phases were combined, dried with anhydrous sodium sulfate, filtered, and concentrated to dryness, so as to obtain 1.3 g of colorless oily product 5-bromo-8-chlorochroman--4-amine. LC-MS: [M+H]⁺=271.1.

### Step D: synthesis of tert-butyl(5-bromo-8-chlorochroman-4-yl)carbamate

At a room temperature, 5-bromo-8-chlorochroman--4-amine (2 g, 7.61 mmol) was dissolved in DCM (30 mL), TEA (2.31 g, 22.86 mmol) and (Boc)₂O (3.32 g, 15.2 mmol) were added, and a mixture was stirred at a room temperature for 48 hours.

At the end of the reaction, water (30 mL) was added to quench the reaction, ethyl acetate (30 mL×3) was used for extraction, organic phases were combined, dried with anhydrous sodium sulfate, filtered, and concentrated to dryness, and the resulting residue was purified by silica gel column chromatography (an eluent: petroleum ether:ethyl acetate=100:1 to 10:1) to obtain 1.2 g (3.31 mmol) of tert-butyl(5-bromo-8-chlorochroman-4-yl)carbamate (yield 43.5%). ¹H NMR (400 MHz, DMSO) δ 7.43 (d, *J*=7.6 Hz, 1H), 7.32 (d, *J*=8.5 Hz, 1H), 7.18 (d, *J*=8.5 Hz, 1H), 4.67 (d, *J*=7.4 Hz, 1H), 4.43 (d, *J*=10.9 Hz, 1H), 4.11 (dd, *J*=22.6, 11.8 Hz, 1H), 2.04-1.82 (m, 2H), 1.41 (s, 9H).

### Step E: synthesis of tert-butyl(5-bromo-8-chlorochroman--4-yl)(tert-butoxycarbonyl)carbamate

At a room temperature, 5-bromo-8-chlorochroman-4-amine (1.2 g, 3.31 mmol) was dissolved in 2-MeTHF (15 mL), DMAP (0.808 g, 6.62 mmol) and (Boc)₂O (1.44 g, 6.62 mmol) were added, and a mixture was heated to 50°C and stirred for 16 hours.

At the end of the reaction, water (20 mL) was added to quench the reaction, ethyl acetate (20 mL×3) was used for extraction, organic phases were combined, dried with anhydrous sodium sulfate, filtered, and concentrated to dryness, and the resulting residue was purified by a reversed-phase column to obtain 1.1 g (2.38 mmol) of tert-butyl(5-bromo-8-chlorochroman--4-yl)(tert-butoxycarbonyl)carbamate (yield 69.8%). ¹H NMR (400 MHz, DMSO) δ 7.34 (d, *J*=8.5 Hz, 1H), 7.17 (d, *J*=8.5 Hz, 1H), 5.30 (t, *J*=4.2 Hz, 1H), 4.41 (qd, *J*=11.1, 5.1 Hz, 2H), 2.19-2.08 (m, 2H), 1.35 (d, *J*=27.6 Hz, 18H).

### Step F: synthesis of tert-butyl(tert-butoxycarbonyl)(8-chloro-5-vinylchroman-4-yl)carbamate

Tert-butyl(5-bromo-8-chlorochroman-4-yl)(tert-butoxycarbonyl)carbamate (1 g, 2.16 mmol), potassium vinyltrifluoroborate (0.43 g, 3.24 mmol), Pd(ppf)Cl₂ (0.158 g, 0.216 mmol), and K₂CO₃ (0.9 g, 6.438 mmol) were dissolved in anhydrous dioxane (20 mL), and a mixture was stirred at 100°C for 16 hours.

At the end of the reaction, water (30 mL) was added to quench the reaction, ethyl acetate (30 mL×3) was used for extraction, organic phases were combined, dried with anhydrous sodium sulfate, filtered, and concentrated to dryness, and the resulting residue was purified by silica gel column chromatography (an eluent: petroleum ether:ethyl acetate=100:1 to 10:1) to obtain 1 g (2.44 mmol) of colorless oily product tert-butyl(tert-butoxycarbonyl)(8-chloro-5-vinylchroman-4-yl)carbamate (crude).

### Step G: synthesis of tert-butyl(tert-butoxycarbonyl)(8-chloro-5-formylchroman-4-yl)carbamate

At a room temperature,
tert-butyl(tert-butoxycarbonyl)(8-chloro-5-vinylchroman-4-yl)carbamate (1 g, 2.44 mmol) was dissolved in THF/H₂O (30 mL/6 mL), K₂OsO₄ (90 mg, 0.244 mmol) and NaIO₄ (2.087 g, 9.785 mmol) were added, and a mixture was stirred at a room temperature for 1 hour.

TLC showed that the reaction was completed, water (30 mL) was added to quench the reaction, ethyl acetate (30 mL×3) was used for extraction, organic phases were combined, dried with anhydrous sodium sulfate, filtered, and concentrated to dryness, and the resulting residue was purified by silica gel column chromatography (an eluent: petroleum ether:ethyl acetate=10:1 to 10:1) to obtain 0.5 g (1.21 mmol) of brown oily product tert-butyl(tert-butoxycarbonyl)(8-formyl-1,2,4-tetrahydronaphthalene-1-yl)carbamate (yield 49.6%).

### Step H: synthesis of ethyl 3-(((4-(bis(tert-butoxycarbonyl amino)-8-chlorochroman-5-yl)methyl)amino)-1H-pyrrole-2-carboxylate

At a room temperature, 3-amino-1H-pyrrole-2-ethyl carboxylate hydrochloride (277 mg, 1.457 mmol) was dissolved in anhydrous MeOH (10 mL), and DIEA (188 mg, 1.457 mmol) and AcOH (87 mg, 1.457 mmol) were added. Tert-butyl(tert-butoxycarbonyl)(8-formyl-1,2,4-tetrahydronaphthalene-1-yl)carbamate (500 mg, 1.214 mmol) was added, and a mixture was stirred at a room temperature for 1 hour. NaBH₃CN (91 mg, 1.457 mmol) was added, and a mixture was stirred at a room temperature for 1 hour.

At the end of the reaction, methanol was removed by rotary evaporation, water (20 mL) was added to quench the reaction, ethyl acetate (20 mL×3) was used for extraction, organic phases were combined, dried with anhydrous sodium sulfate, filtered, and concentrated to dryness, and the resulting residue was purified by a reversed-phase column to obtain 300 mg (0.545 mmol) of ethyl 3-(((4-(bis(tert-butoxycarbonyl amino)-8-chlorochroman-5-yl)methyl)amino)-1H-pyrrole-2-carboxylate (yield 44.9%).
LC-MS: [M+H]⁺=550.3. ¹H NMR (400 MHz, DMSO) δ 10.80 (s, 1H), 7.32 (d, *J*=8.3 Hz, 1H), 6.86 (d, *J*=8.3 Hz, 1H), 6.72 (t, *J*=3.0 Hz, 1H), 5.72 (s, 1H), 5.54-5.40 (m, 2H), 4.47 (dt, *J*=13.5, 6.8 Hz, 1H), 4.32-4.24 (m, 2H), 4.23-4.09 (m, 3H), 2.15 (d, *J*=5.6 Hz, 2H), 1.28 (s, 7H).

### Step I:

### tert-butyl(tert-butoxycarbonyl)(8-chloro-5-((4-oxo-2-thio-2,3,4,5-tetrahydro-1H-pyrrol o[3,2-d]pyrimidin-1-yl)methyl)pyran-4-yl)carbamate

At a room temperature, ethyl 3-(((4-(bis(tert-butoxycarbonyl amino)-8-chlorochroman-5-yl)methyl)amino)-1H-pyrrole-2-carboxylate (300 mg, 0.545 mmol) was dissolved in MeOH (10 mL), benzoyl isothiocyanate (107 mg, 0.654 mmol) was added, a mixture was stirred at a room temperature for 1 hour, then Cs₂CO₃ (355 mg, 1.091 mmol) was added, and a mixture reacted at 65°C for 3 hours.

At the end of the reaction, a solvent was concentrated to dryness, and the resulting residue was purified using a reaction column to obtain 189 mg (0.336 mmol) of tert-butyl(tert-butoxycarbonyl)(8-chloro-5-((4-oxo-2-thio-2,3,4,5-tetrahydro-1H-pyrrolo[3,2 -d]pyrimidin-1-yl)methyl)pyran-4-yl)carbamate (yield: 61.7%). LC-MS: [M+H]⁺=563.2. ¹H NMR (400 MHz, DMSO) δ 12.51 (s, 1H), 12.38 (s, 1H), 7.33 (t, *J*=2.9 Hz, 1H), 7.23 (d, *J*=8.4 Hz, 1H), 6.24 (d, *J*=8.4 Hz, 1H), 5.94 (s, 1H), 5.65 (t, *J*=5.2 Hz, 2H), 5.28 (d, *J*=16.5 Hz, 1H), 4.51 (dt, *J*=12.1, 6.0 Hz, 1H), 4.38-4.27 (m, 1H), 2.29 (t, *J*=12.3 Hz, 2H), 1.33 (s, 18H).

### Step J: synthesis of 1-((4-amino-8-chlorochroman-5-yl)methyl)-2-thio-1,2,3,5-tetrahydro-4H-pyrrolo[3,2-d] pyrimidin-4-one

At a room temperature,
tert-butyl(tert-butoxycarbonyl)(8-chloro-5-((4-oxo-2-thio-2,3,4,5-tetrahydro-1H-pyrrolo[3,2 -d]pyrimidin-1-yl)methyl)pyran-4-yl)carbamate (240 mg, 0.426 mmol) was dissolved in DCF (10 mL), hydrochloric acid (1.1 mg, 4.26 mmol, 4 M in dioxane) was added, and a mixture was stirred at a room temperature for 3 hours.

At the end of the reaction, a reaction solution was filtered, and a filter cake was washed with DCM (5 mL) and dried to obtain 150 mg (0.376 mmol) of 1-(8-amino-5,6,7,8-tetrahydronaphthalene-1-yl)methyl)-2-thio-1,2,3,5-tetrahydro-4H-pyrrol o [3,2-d]pyrimidin-4-one hydrochloride (yield: 88.2%). LC-MS: [M+H]⁺=362.1. ¹H NMR (400 MHz, DMSO) δ 12.53 (s, 1H), 12.40 (s, 1H), 8.76 (s, 3H), 7.36 (dd, *J*=9.8, 5.5 Hz, 2H), 6.27 (d, *J*=8.4 Hz, 1H), 6.19 (s, 1H), 5.95 (d, *J*=16.7 Hz, 1H), 5.71 (d, *J*=16.7 Hz, 1H), 4.95 (s, 1H), 4.57 (dd, *J*=11.4, 4.0 Hz, 1H), 4.39 (t, *J*=11.8 Hz, 1H), 2.42 (d, *J*=13.6 Hz, 1H), 2.29-2.15 (m, 1H).

### Example 6

### 1-((4-aminochroman-5-yl)methyl)-2-thio-1,2,3,5-tetrahydro-4H-pyrrolo[3,2-d]|pyrimidi n-4-one, compound 6A and compound 6B

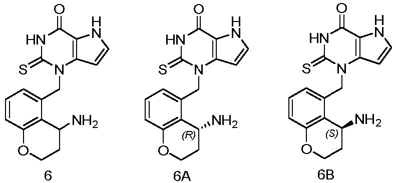

### Synthetic route:

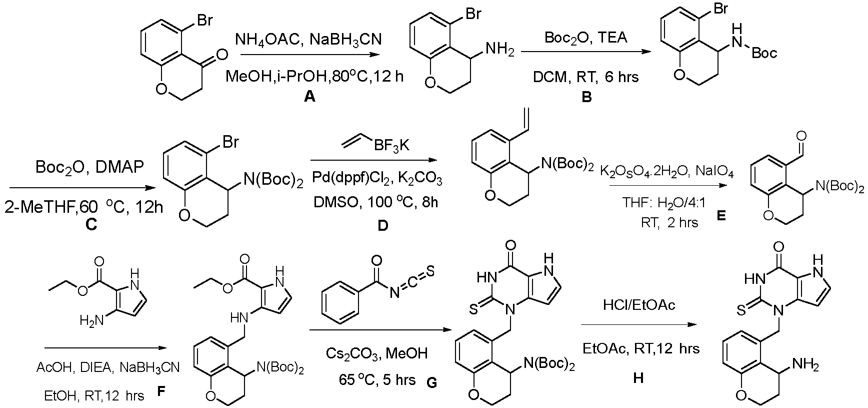

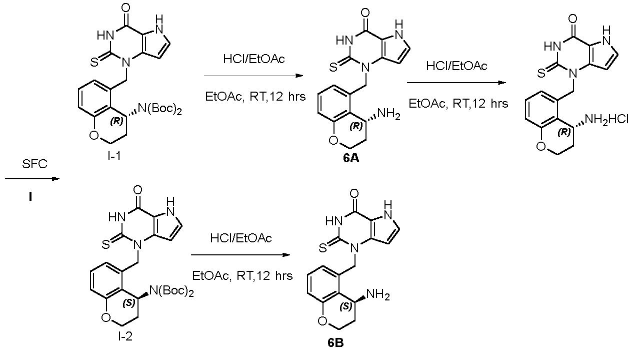

### Step A: synthesis of 5-bromochroman-4-amine

5-bromo-4-benzodihydropyranone (4.4 g, 19.37 mmol) was dissolved in anhydrous methanol (44 mL) and isopropanol (55 mL), ammonium acetate (29.87 g, 387 mmol) and sodium cyanoborohydride (6.08 g, 96.85 mmol) were added, and a mixture was refluxed at 80°C for 12 hours.

At the end of the reaction, a solvent in the solution was subjected to spin drying, 1 mol of sodium hydroxide solution was used to adjust a pH to 10, ethyl acetate (30 mL×2) was used for extraction, organic phases were combined and dried with anhydrous sodium sulfate, filtered, and concentrated to dryness, and the resulting residue was purified using silica gel column chromatography (an eluent: petroleum ether/ethyl acetate=0/1) to obtain 3 g of white solid 5-bromo-4-benzodihydropyran-amine (yield: 68.0%). ¹H NMR (400 MHz, DMSO) δ 7.16-7.01 (m, 2H), 6.86-6.76 (m, 1H), 4.32-4.17 (m, 2H), 4.09-3.98 (m, 1H), 1.96-1.77 (m, 2H).

### Step B: synthesis of tert-butyl(5-bromo-4-benzodihydropyran-amine)carbamate

5-bromo-4-benzodihydropyran-amine (3.0 g, 13.2 mmol), di-tert-butyl dicarbonate (3.15 g, 14.5 mmol), and triethylamine (4.0 g, 39.6 mmol) were dissolved in dichloromethane (30 mL), and a mixture reacted at a room temperature for 6 hours.

20 mL of water was added to a reaction solution, dichloromethane (20 mL×2) was used for extraction, and organic phases were combined, washed with a NaHCO₃ aqueous solution, dried with anhydrous sodium sulfate, filtered, and subjected to spin drying. The resulting residue was purified by silica gel column chromatography (an eluent: petroleum ether/ethyl acetate=10/1) to obtain 3.8 g of tert-butyl(5-bromo-4-benzodihydropyran-amine)carbamate (yield: 88.3%). LCMS: m/z (ESI), [M+Na]⁺=350.0; ¹H NMR (400 MHz, DMSO) δ 7.41 (d, J=7.6 Hz, 1H), 7.16-7.09 (m, 2H), 6.81 (dd, J=8.0, 1.2 Hz, 1H), 4.63 (s, 1H), 4.25 (d, J=10.8 Hz, 1H), 4.04 (dd, J=17.2, 6.4 Hz, 1H), 1.98-1.78 (m, 2H), 1.42 (s, 9H).

### Step C: synthesis of tert-butyl(5-bromo-4-benzodihydropyran)(tert-butoxycarbonyl)carbamate

Tert-butyl(5-bromo-4-benzodihydropyran-amine)carbamate (3.8 g, 11.58 mmol), di-tert-butyl dicarbonate (5.05 g, 23.16 mmol), and 4-dimethylaminopyridine (2.83 g, 23.16 mmol) were dissolved in dimethyltetrahydrofuran (20 mL), and a mixture reacted at 60°C for 12 hours.

At the end of the reaction, the mixture was cooled to a room temperature and subjected to spin drying, 20 mL of water was added, ethyl acetate (30 mL×2) was used for extraction, and organic phases were combined, washed with a NaHCO₃ aqueous solution, dried with anhydrous sodium sulfate, filtered, and subjected to spin drying. The resulting residue was purified by silica gel column chromatography (an eluent: petroleum ether/ethyl acetate=10/1) to obtain 3.5 g of tert-butyl(5-bromo-4-benzodihydropyran)(tert-butoxycarbonyl)carbamate (yield: 71.4%).
¹H NMR (400 MHz, DMSO) δ 7.19-7.08 (m, 2H), 6.87-6.78 (m, 1H), 5.26 (t, J=4.2 Hz, 1H), 4.37-4.32 (m, 1H), 4.27-4.15 (m, 1H), 2.16-2.00 (m, 2H), 1.34 (s, 18H).

### Step D: synthesis of tert-butyl(5-vinyl-4-benzodihydropyran)(tert-butoxycarbonyl)carbamate

Tert-butyl(5-bromo-4-benzodihydropyran)(tert-butoxycarbonyl)carbamate (3.5 g, 8.17 mmol), potassium vinyltrifluoroborate (1.42 g, 10.6 mmol), 1,1'-bis(diphenylphosphine)ferrocene palladium chloride (1.18 g, 1.63 mmol), and potassium carbonate (3.38 g, 24.5 mmol) were dissolved in dimethyl sulfoxide (30 mL), and a mixture reacted at 100°C under nitrogen protection for 12 hours.

At the end of the reaction, the mixture was cooled to a room temperature, a reaction solution was filtered through diatomaceous earth, filtrate was collected, then 50 mL of water was added, ethyl acetate (40 mL×3) was used for extraction, and organic phases were combined, washed with a NaCl aqueous solution, dried with anhydrous sodium sulfate, filtered, and subjected to spin drying. The resulting residue was purified by silica gel column chromatography (an eluent: petroleum ether/ethyl acetate=10/1) to obtain 1.8 g of yellow oily tert-butyl(5-vinyl-4-benzodihydropyran)(tert-butoxycarbonyl)carbamate (yield: 58.8%). ¹H NMR (400 MHz, DMSO) δ 7.15-7.06 (m, 1H), 7.06 (d, J=7.2 Hz, 1H), 6.76-6.69 (m, 2H), 5.65 (dd, J=17.2, 1.2 Hz, 1H), 5.41 (t, J=5.2 Hz, 1H), 5.30-5.21 (m, 1H), 4.36 (dd, J=7.2, 3.6 Hz, 1H), 4.17-4.08 (m, 1H), 2.17-2.02 (m, 3H), 1.26 (s, 18H).

### Step E: synthesis of tert-butyl(5-formyl-4-benzodihydropyran)(tert-butoxycarbonyl)carbamate

Tert-butyl(5-vinyl-4-benzodihydropyran)(tert-butoxycarbonyl)carbamate (1.0 g, 2.67 mmol) was dissolved in a mixed solution of tetrahydrofuran (16 mL) and water (4 mL), potassium osmate (98.2 mg, 0.267 mmol) was added to a reaction solution, the reaction solution was stirred at a room temperature for 30 minutes, then sodium periodate (2.28 g, 10.68 mmol) was added to the reaction solution, and the reaction solution reacted at a room temperature for 2 hours.

At the end of the reaction, 20 mL of water was added to the reaction solution, ethyl acetate (20 mL×2) was used for extraction, and organic phases were combined, dried with anhydrous sodium sulfate, filtered, and subjected to spin drying. The resulting residue was purified by silica gel column chromatography (an eluent: petroleum ether/ethyl acetate=10/1) to obtain 600 mg of yellow oily tert-butyl(5-formyl-4-benzodihydropyran)(tert-butoxycarbonyl)carbamate (yield: 60.0%). LCMS: m/z (ESI), [M+Na]⁺=400.1; ¹H NMR (400 MHz, DMSO) δ 10.02 (s, 1H), 7.42-7.39 (m, 2H), 7.14-7.01 (m, 1H), 5.89 (t, J=5.6 Hz, 1H), 4.43-4.37 (m, 1H), 4.23-4.18 (m, 1H), 2.2-2.12 (m, 2H), 1.26 (s, 18H).

### Step F: synthesis of ethyl 3-(((4-(bis(tert-butoxycarbonyl)amino)pyran-5-yl)methyl)amino)-1H-pyrrole-2-carbox ylate

3-amino-2-ethoxycarbonylpyrrole hydrochloride (333 mg, 1.75 mmol) was dissolved in 6 mL of anhydrous ethanol, then N,N-diisopropylethylamine (190 mg, 1.75 mmol) and glacial acetic acid (270 mg, 4.74 mmol) were added to a mixture, the mixture was stirred at a room temperature for 10 minutes, then tert-butyl(5-formyl-4-benzodihydropyran)(tert-butoxycarbonyl)carbamate (600 mg, 1.58 mmol) was added to the mixture, and stirred at a room temperature for 2 hours, then sodium cyanoborohydride (180 mg, 3.16 mmol) was added to the mixture, and the mixture reacted at a room temperature for 12 hours.

At the end of the reaction, the anhydrous ethanol in the reaction solution was subjected to spin drying. 10 mL of water and ethyl acetate (20 mL×2) were used for extraction, and organic phases were combined, dried with anhydrous sodium sulfate, filtered, and subjected to spin drying. The resulting residue was purified using a reversed-phase column (an eluent: 0.1% ammonia water solution) to obtain 450 mg of ethyl 3-(((4-(bis(tert-butoxycarbonyl)amino)pyran-5-yl)methyl)amino)-1H-pyrrole-2-carboxylate (yield: 81.9%). ¹H NMR (400 MHz, DMSO) δ 10.78 (s, 1H), 7.13 (t, J=8.0 Hz, 1H), 6.86 (d, J=7.6 Hz, 1H), 6.73-6.67 (m, 2H), 5.63 (s, 1H), 5.52 (t, J=2.4 Hz, 1H), 5.43 (t, J=4.4 Hz, 1H), 4.47-4.34 (m, 1H), 4.35-4.21 (m, 1H), 4.24-4.08 (m, 4H), 2.13-2.09 (m, 2H), 1.26 (m, 18H), 1.18 (dd, J=9.2, 5.2 Hz, 3H).

### Step G: synthesis of tert-butyl(tert-butoxycarbonyl)(5-((4-oxo-2-thio-2,3,4,5-tetrahydro-1H-pyrrolo[3,2-d]p yrimidin-1-yl)methyl)chroman-4-yl)carbamate

Ethyl3-(((4-(bis(tert-butoxycarbonyl)amino)pyran-5-yl)methyl)amino)-1H-pyrrole-2-ca rboxylate (450 mg, 0.87 mmol) and benzoyl isothiocyanate (171 mg, 1.05 mmol) ware dissolved in methanol (10 mL), a mixture was stirred at a room temperature for 3 hours, then cesium carbonate (567 mg, 1.74 mmol) was added, and a reaction solution reacted at 65°C for 2 hours.

At the end of the reaction, the anhydrous methanol in the reaction solution was subjected to spin drying. 20 mL of water and ethyl acetate (20 mL×2) were used for extraction, and organic phases were combined, dried with anhydrous sodium sulfate, filtered, and subjected to spin drying. The resulting residue was purified using a reversed-phase column (an eluent: 0.1% ammonium bicarbonate solution) to obtain 400 mg of tert-butyl(tert-butoxycarbonyl)(5-((4-oxo-2-thio-2,3,4,5-tetrahydro-1H-pyrrolo[3,2-d]pyrimi din-1-yl)methyl)chroman-4-yl)carbamate (yield: 86.9%). LCMS: m/z (ESI), [M+H]⁺=529.0; ¹H NMR (400 MHz, DMSO) δ 7.33 (t, J=2.8 Hz, 1H), 7.04 (t, J=8.0 Hz, 1H), 6.71 (d, J=8.0 Hz, 1H), 6.24 (d, J=7.6 Hz, 1H), 5.91 (t, J=2.4 Hz, 1H), 5.71-5.67 (m, 1H), 5.60 (t, J=5.2 Hz, 1H), 5.35 (d, J=16.4 Hz, 1H), 4.44-4.41 (m, 1H), 4.21-4.17 (m, 1H), 2.26-2.22 (m, 1H), 1.32 (s, 18H).

### Step H: synthesis of 1-((4-aminochroman-5-yl)methyl)-2-thio-1,2,3,5-tetrahydro-4H-pyrrolo[3,2-d]|pyrimidi n-4-one

Tert-butyl(tert-butoxycarbonyl)(5-((4-oxo-2-thio-2,3,4,5-tetrahydro-1H-pyrrolo[3,2-d]p yrimidin-1-yl)methyl)chroman-4-yl)carbamate (200 mg, 0.37 mmol) was dissolved in ethyl acetate (10 mL), an ethyl acetate hydrochloride solution (10 mL) was added to a reaction solution, and the reaction solution was stirred at a room temperature for 12 hours. The reaction solution was subjected to spin drying. Then a sodium bicarbonate aqueous solution was used to adjust a pH of the solution to 8, ethyl acetate (15 mL×2) was used for extraction, and organic phases were combined, dried with anhydrous sodium sulfate, filtered, and subjected to spin drying. The resulting residue was purified using a reversed-phase column (an eluent: 0.1% ammonia water solution) to obtain 93.83 mg of 1-((4-aminochroman-5-yl)methyl)-2-thio-1,2,3,5-tetrahydro-4H-pyrrolo[3,2-d]pyrimidin-4-o ne (yield: 68.4%). LCMS: m/z (ESI), [M+H]⁺=329.1; ¹H NMR(400 MHz, DMSO-d6) δ 7.29 (d, J=2.9 Hz, 1H), 6.96 (t, J=7.9 Hz, 1H), 6.64(d,J=8.1 Hz, 1H), 6.21-6.15 (m, 2H), 5.97 (d, J=16.4 Hz, 1H), 5.83 (d, J=16.4 Hz, 1H), 4.34-4.18 (m, 2H), 4.15-4.05 (m, 1H), 2.07-1.95 (m, 1H), 1.87-1.79 (m, 1H).

### Step I: synthesis of compounds I-1 and I-2

Tert-butyl(tert-butoxycarbonyl)(5-((4-oxo-2-thio-2,3,4,5-tetrahydro-1H-pyrrolo[3,2-d]p yrimidin-1-yl)methyl)chroman-4-yl)carbamate (200 mg, 0.38 mmol) was fed to SFC for separation (DAICELCHIRALCEL^{®}OZ column (250*25 mm 10 µm), Supercritical CO₂ and MEOH (+0.1% 7.0mol/l Ammonia in MEOH) were used as mobile phases for chiral separation preparation, so as to obtain an isomer I-1 (RT=4.18 min, 70 mg, yield=34%, ee>99%) and I-2 (RT=5.16 min, 65 mg, yield=32%, ee>99%).

### Preparation of compound 6A:

A compound I-1 (200 mg, 0.37 mmol) was dissolved in ethyl acetate (10 mL), an ethyl acetate hydrochloride solution (10 mL) was added to a reaction solution, and the reaction solution was stirred at a room temperature for 12 hours. The reaction solution was subjected to spin drying. Then a sodium bicarbonate aqueous solution was used to adjust a pH of the solution to 8, ethyl acetate (15 mL×2) was used for extraction, and organic phases were combined, dried with anhydrous sodium sulfate, filtered, and subjected to spin drying. The resulting residue was purified using a reversed-phase column (an eluent: 0.1% ammonia water solution) to obtain 96.5 mg of compound 6A(R)-1-((4-aminochroman-5-yl)methyl)-2-thio-1,2,3,5-tetrahydro-4H-pyrrolo[3,2-d]pyrimi din-4-one (yield: 77.8%). ee> 99%. LCMS: m/z (ESI), [M+H]⁺=329.1; ¹H NMR (400 MHz, DMSO-*d*₆) δ 7.28 (d, *J*=2.8 Hz, 1H), 6.95 (t, *J*=7.9 Hz, 1H), 6.64 (d, *J*=8.1 Hz, 1H), 6.21-6.13 (m, 2H), 5.97 (d, *J*=16.4 Hz, 1H), 5.83 (d, *J*=16.4 Hz, 1H), 4.34-4.17 (m, 2H), 4.11 (s, 1H), 2.07-1.94 (m, 1H), 1.88-1.76 (m, 1H).

### Preparation of compound 6A hydrochloride:

(R)-tert-butyl(tert-butoxycarbonyl)(5-((4-oxo-2-thio-2,3,4,5-tetrahydro-1H-pyrrolo[3 ,2-d]pyrimidin-1-yl)methyl)chroman-4-yl)carbamate (200 mg, 0.37 mmol) was dissolved in ethyl acetate (10 mL), an ethyl acetate hydrochloride solution (4M, 10 mL) was added to a reaction solution, and the reaction solution was stirred at a room temperature for 12 hours. The reaction solution was filtered and washed with ethyl acetate to obtain 94 mg of white solid (R)-1-((4-aminochroman-5-yl)methyl)-2-thio-1,2,3,5-tetrahydro-4H-pyrrolo[3,2-d]pyrimidin -4-one hydrochloride (yield: 68%). LCMS: m/z (ESI), [M+H]+=329.1. ¹H NMR (400 MHz, DMSO-d6) δ 12.52 (s,1H),12.38 (s,1H), 8.63 (s,3H), 7.36 (t,J=3.0 Hz, 1H), 7.15 (t, J=8.0 Hz, 1H), 6.77 (d,J=8.2 Hz, 1H), 6.26 (d,J=7.7Hz,1H), 6.16 (t,J=2.5Hz,1H), 5.93 (d,J=16.6 Hz, 1H), 5.71 (d,J=16.6 Hz, 1H), 4.89 (s,1H), 4.45-4.36 (m,1H), 4.34-4.25 (m,1H), 2.39-2.31 (m,1H), 2.26-2.14 (m,1H).

The schematic structural diagram of a single crystal of compound 6A hydrochloride is as shown in FIG. 1, and the specific crystal parameters are as follows:

| **Crystal system** | **orthorhombic** |
|---|---|
| **Space group** | P2₁2₁2₁ |
| **a/Å** | 7.40910 (10) |
| **b/Å** | 12.7287 (2) |
| **c/Å** | 17.0243 (3) |
| **α/°** | 90 |
| **β/°** | 90 |
| ***γ*/°** | 90 |
| **Cell Volume/Å³** | 1605.53 (4) |
| **Z** | 4 |
| **Crystal Density calc g/cm³** | 1.509 |
| **Crystal F(000)** | 760.0 |
| **Absorption Coefficient µ/mm⁻¹** | 3.478 |
| **Cell Measurement Temperature/K** | 293 (2) |
| **20 range for data collection/°** | 8.674 to 133.198 |
| **Index ranges** | -8 ≤ h ≤ 7, -15 ≤ k ≤ 13, -20 ≤ 1 ≤ 19 |
| **Reflections collected** | 16011/2843[Rᵢₙₜ=0.0464] |
| **Goodness-of-fit on F²** | 1.026 |
| **Final R indexes [I>=2σ (I)]** | R₁=0.0272, wR₂=0.0702 |
| **Final R indexes [all data]** | R₁=0.0286, wR₂=0.0710 |
| **Largest diff. peak/hole / e Å⁻³** | 0.33/-0.18 |
| **Flack parameter** | -0.003 (7) |

### Preparation of compound 6B:

A compound I-2 (200 mg, 0.37 mmol) was dissolved in ethyl acetate (10 mL), an ethyl acetate hydrochloride solution (10 mL) was added to a reaction solution, and the reaction solution was stirred at a room temperature for 12 hours. The reaction solution was subjected to spin drying. Then a sodium bicarbonate aqueous solution was used to adjust a pH of the solution to 8, ethyl acetate (15 mL×2) was used for extraction, and organic phases were combined, dried with anhydrous sodium sulfate, filtered, and subjected to spin drying. The resulting residue was purified using a reversed-phase column (an eluent: 0.1% ammonia water solution) to obtain 93.6 mg of compound 6B(S)-1-((4-aminochroman-5-yl)methyl)-2-thio-1,2,3,5-tetrahydro-4H-pyrrolo[3,2-d]pyrimi din-4-one (yield: 75.3%). ee> 99%. LCMS: m/z (ESI), [M+H]⁺=329.1.

Synthesis steps of compound 6B hydrochloride are the same as the above steps. LCMS: m/z (ESI), [M+H]⁺=329.1. ¹H NMR (400 MHz, DMSO-d6) δ 12.52 (s, 1H), 12.38 (s, 1H), 8.63 (s, 3H), 7.36 (t, J=3.0 Hz, 1H), 7.15 (t, J=8.0 Hz, 1H), 6.77 (d, J=8.2 Hz, 1H), 6.26 (d, J=7.7 Hz, 1H), 6.16 (t, J=2.5 Hz, 1H), 5.93 (d, J=16.6 Hz, 1H), 5.71 (d, J=16.6 Hz, 1H), 4.89 (s, 1H), 4.45-4.36 (m, 1H), 4.34-4.25 (m, 1H), 2.39-2.31 (m, 1H), 2.26-2.14 (m, 1H).

### Example 7

### 1-((8-amino-5,6,7,8-tetrahydronaphthalene-1-yl)methyl)-2-thio-1,2,3,5-tetrahydro-4H-pyrrolo[3,2-d]pyrimidin-4-one, compound 7A and compound 7B

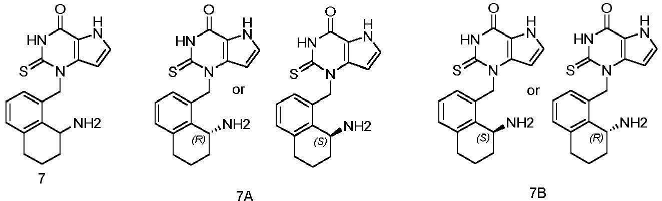

### Synthetic route:

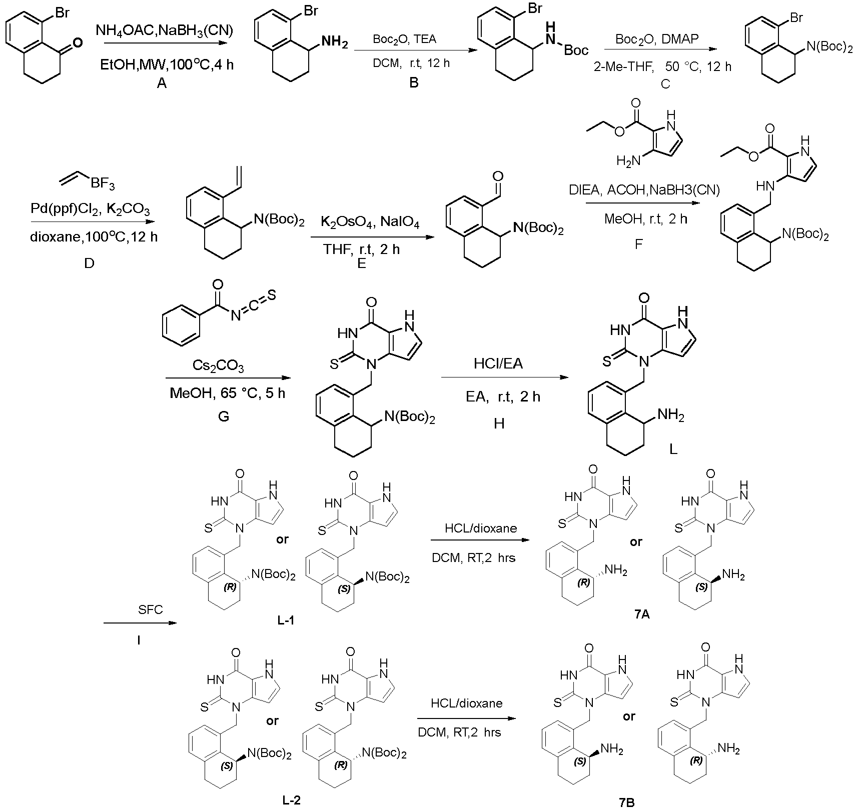

### Step A: synthesis of 8-bromo-1,2,3,4-tetrahydronaphthalene-1-amine

At a room temperature, 8-bromo-3,4-dihydronaphthalene-1(2H)-one (500 mg, 2.22 mmol) was dissolved in ETOH (10 mL), NH₄OAC (1.7 g, 22.22 mmol) and NaBH₃CN (700 mg, 11.11 mmol) were added, and a mixture was stirred at 100°C for 4 hours.

At the end of the reaction, ethanol was removed by spin drying, water (50 mL) was added to quench the reaction, dichloromethane (50 mL×3) was used for extraction, organic phases were combined, dried with anhydrous sodium sulfate, filtered, and concentrated to dryness, so as to obtain 330 mg (1.47 mmol) of white solid product 8-bromo-1,2,3,4-tetrahydronaphthalene-1-amine (yield: 66.0%). ¹H NMR (400 MHz, DMSO-d₆) δ 7.40 (d, J=7.3 Hz, 1H), 7.16-6.94 (m, 2H), 4.08-3.96 (m, 1H), 2.88-2.56 (m, 2H), 2.05-1.79 (m, 4H), 1.74-1.56 (m, 2H).

### Step B: synthesis of tert-butyl(8-bromo-1,2,3,4-tetrahydronaphthalene-1-yl)carbamate

At a room temperature, 8-bromo-1,2,3,4-tetrahydronaphthalene-1-amine (300 mg, 1.46 mmol) was dissolved in DCM (30 mL), TEA (440 mg, 4.38 mmol) and (BOC)₂O (640 mg, 2.92 mmol) were added, and a mixture was stirred at a room temperature for 12 hours.

At the end of the reaction, water (40 mL) was added to quench the reaction, dichloromethane (30 mL×3) was used for extraction, organic phases were combined, dried with anhydrous sodium sulfate, filtered, and concentrated to dryness, and the resulting residue was purified by silica gel column chromatography (an eluent: petroleum ether:ethyl acetate=10:1) to obtain 370 mg (1.14 mmol) of yellow liquid product tert-butyl(8-bromo-1,2,3,4-tetrahydronaphthalene-1-yl)carbamate (yield 78.0%). ¹H NMR (400 MHz, DMSO-d₆) δ 7.42 (t, J=4.5 Hz, 1H), 7.22-7.04 (m, 3H), 4.70 (d, J=7.9 Hz, 1H), 2.80-2.58 (m, 2H), 1.93-1.54 (m, 4H), 1.39 (s, 9H).

### Step C: Synthesis of methyl tert-butyl(8-bromo-1,2,3,4-tetrahydronaphthalene-1-yl)(tert-butoxycarbonyl)carba mate

(8-bromo-1,2,3,4-tetrahydronaphthalene-1-yl)carbamate (370 mg, 1.14 mmol) was dissolved in 2-methyltetrahydrofuran, DMAP (280 mg, 2.28 mmol) and (BOC)₂O (500 mg, 2.21 mmol) were added, and a mixture was stirred at 50°C for 12 hours.

At the end of the reaction, water (40 mL) was added to quench the reaction, ethyl acetate (30 mL×3) was used for extraction, organic phases were combined, dried with anhydrous sodium sulfate, filtered, and concentrated to dryness, and the resulting residue was purified by silica gel column chromatography (an eluent: petroleum ether:ethyl acetate=10:1) to obtain 220 mg (0.52 mmol) of colorless liquid product methyl tert-butyl(8-bromo-1,2,3,4-tetrahydronaphthalene-1-yl)(tert-butoxycarbonyl)carbamate (yield 45.8%). ¹H NMR (400 MHz, DMSO-d₆) δ 7.42 (t, J=4.5 Hz, 1H), 7.22-6.95 (m, 3H), 4.70 (d, J=7.9 Hz, 1H), 2.82-2.56 (m, 2H), 1.96-1.53 (m, 4H), 1.39 (s, 18H).

### Step D: synthesis of tert-butyl(tert-butoxycarbonyl)(8-vinyl-1,2,3,4-tetrahydronaphthalene-1-yl)carbamate

Methyl tert-butyl(8-bromo-1,2,3,4-tetrahydronaphthalene-1-yl)(tert-butoxycarbonyl)carbamate (220 mg, 0.52 mmol), potassium vinyltrifluoroborate (100 mg, 0.78 mmol), Pd(ppf)Cl₂ (36 mg, 0.05 mmol), and K₂CO₃ (200 mg, 1.56 mmol) were dissolved in anhydrous dioxane (10 mL), and a mixture was stirred at 100°C for 12 hours.

At the end of the reaction, water (40 mL) was added to quench the reaction, ethyl acetate (30 mL×3) was used for extraction, organic phases were combined, dried with anhydrous sodium sulfate, filtered, and concentrated to dryness, and the resulting residue was purified by silica gel column chromatography (an eluent: petroleum ether:ethyl acetate=10:1) to obtain 30 mg (0.08 mmol) of colorless liquid product tert-butyl(tert-butoxycarbonyl)(8-vinyl-1,2,3,4-tetrahydronaphthalene-1-yl)carbamate (yield 15.7%). ¹H NMR (400 MHz, DMSO-d₆) δ 11.01-10.45 (m, 1H), 7.24-6.91 (m, 3H), 6.72 (s, 1H), 5.75 (s, 1H), 5.48 (s, 1H), 4.41-4.05 (m, 4H), 2.89-2.63 (m, 1H), 2.01 (s, 3H), 1.62-1.55 (m, 1H), 1.25 (s, 19H), 0.89-0.67 (m, 2H).

### Step E: synthesis of tert-butyl(tert-butoxycarbonyl)(8-formyl-1,2,4-tetrahydronaphthalene-1-yl)carbamate

At a room temperature,
tert-butyl(tert-butoxycarbonyl)(8-vinyl-1,2,3,4-tetrahydronaphthalene-1-yl)carbamate (30 mg, 0.08 mmol) was dissolved in anhydrous THF (5 mL), K₂OsO₄ (5 mg, 0.01 mmol) was added, a mixture was stirred at a room temperature for 1 hour, then NaIO₄ (136 mg, 0.64 mmol) was added, and a mixture was stirred at a room temperature for 1 hour.

At the end of the reaction, water (40 mL) was added to quench the reaction, dichloromethane (30 mL×3) was used for extraction, organic phases were combined, dried with anhydrous sodium sulfate, filtered, and concentrated to dryness, and the resulting residue was purified by silica gel column chromatography (an eluent: petroleum ether:ethyl acetate=10:1) to obtain 30 mg (0.08 mmol) of white solid product tert-butyl(tert-butoxycarbonyl)(8-formyl-1,2,4-tetrahydronaphthalene-1-yl)carbamate (yield 98.0%). ¹H NMR (400 MHz, DMSO-d₆) δ 10.06 (s, 1H), 7.61(dd, J=7.2, 1.8Hz, 1H), 7.48-6.94 (m, 2H), 5.91 (t, d=6.6Hz, 1H), 2.88-2.64 (m, 2H), 2.23-1.90 (m, 3H), 1.75-1.56 (m, 2H), 1.25 (s, 18H).

### Step F: synthesis of ethyl 3-((8-(bis(tert-butoxycarbonyl)amino)-5,6,7,8-tetrahydronaphthalene-1-yl)methyl)ami no)-1H-pyrrole-2-carboxylate

At a room temperature,
tert-butyl(tert-butoxycarbonyl)(8-formyl-1,2,4-tetrahydronaphthalene-1-yl)carbamate (30 mg, 0.08 mmol) was dissolved in anhydrous MeOH (5 mL), and DIEA (20 mg, 0.1 mmol) and ACOH (6 mg, 0.1 mmol) were added. After a mixture was stirred at a room temperature for 5 minutes, 3-amino-1H-pyrrole-2-ethyl carboxylate (14 mg, 0.1 mmol) was added. After a mixture was stirred at a room temperature for 1 hour, NaBH₃(CN) (6 mg, 0.1 mmol) was added, and a mixture was stirred at a room temperature for 1 hour.

At the end of the reaction, methanol was removed by rotary evaporation, water (40 mL) was added to quench the reaction, dichloromethane (30 mL×3) was used for extraction, organic phases were combined, dried with anhydrous sodium sulfate, filtered, and concentrated to dryness, and the resulting residue was purified by silica gel column chromatography (an eluent: acetonitrile:water=5:1) to obtain 40 mg (0.08 mmol) of white solid product ethyl 3-((8-(bis(tert-butoxycarbonyl)amino)-5,6,7,8-tetrahydronaphthalene-1-yl)methyl)amino)-1 H-pyrrole-2-carboxylate (yield 97.5%). LC-MS: [M+H]⁺=514.3. ¹H NMR (400 MHz, DMSO-d₆) δ 10.88-10.08 (m, 1H), 7.20-6.93 (m, 3H), 6.72 (s, 1H), 5.75 (s, 1H), 5.48 (s, 2H), 4.35-4.11 (m, 4H), 2.87-2.63 (m, 2H), 2.01 (s, 3H), 1.62-1.48 (m, 1H), 1.21 (d, J=31.1Hz, 19H), 0.88-0.71 (m, 2H).

### Step G:

### tert-butyl(tert-butoxycarbonyl)(8-((4-oxo-2-thio-2,3,4,5-tetrahydro-1H-pyrrolo[3,2-d]p yrimidin-1-yl)methyl)-1,2,3,4-tetrahydronaphthalene-1-yl)carbamate

At a room temperature, ethyl 3-((8-(bis(tert-butoxycarbonyl)amino)-5,6,7,8-tetrahydronaphthalene-1-yl)methyl)amino)-1 H-pyrrole-2-carboxylate (40 mg, 0.08 mmol) was dissolved in MeOH (5 mL), benzoyl isothiocyanate (17 mg, 0.1 mmol) was added, a mixture was stirred at a room temperature for 2 hour, then Cs₂CO₃ (104 mg, 0.32 mmol) was added, and a mixture was stirred at 65°C for 5 hours.

At the end of the reaction, water (40 mL) was added to quench the reaction, dichloromethane (30 mL×3) was used for extraction, organic phases were combined, dried with anhydrous sodium sulfate, filtered, and concentrated to dryness, and the resulting residue was purified by silica gel column chromatography (an eluent: acetonitrile:water=5:1) to obtain 40 mg (0.08 mmol) of white solid product tert-butyl(tert-butoxycarbonyl)(8-((4-oxo-2-thio-2,3,4,5-tetrahydro-1H-pyrrolo[3,2-d]pyrimi din-1-yl)methyl)-1,2,3,4-tetrahydronaphthalene-1-yl)carbamate (yield 95.2%). LC-MS: [M+H]⁺=527.2. ¹H NMR (400 MHz, DMSO-d₆) δ 12.41 (d, J=65.8Hz, 2H), 7.33 (s, 1H), 7.03 (d, J=7.5Hz, 2H), 6.62-6.36 (m, 1H), 5.91-5.57 (m, 3H), 5.47-5.26 (m, 1H), 2.83-2.68 (m, 2H), 2.01 (s, 3H), 2.07 (s, 3H), 1.69-1.59 (m, 2H), 1.29 (s, 18H).

### Step H: synthesis of 1-((8-amino-5,6,7,8-tetrahydronaphthalene-1-yl)methyl)-2-thio-1,2,3,5-tetrahydro-4H-pyrrolo[3,2-d]pyrimidin-4-one

At a room temperature,
tert-butyl(tert-butoxycarbonyl)(8-((4-oxo-2-thio-2,3,4,5-tetrahydro-1H-pyrrolo[3,2-d]pyrimi din-1-yl)methyl)-1,2,3,4-tetrahydronaphthalene-1-yl)carbamate (40 mg, 0.08 mmol) was dissolved in ethyl acetate (10 mL), an ethyl acetate hydrochloride solution (10 mL) was added, and a mixture was stirred at a room temperature for 2 hours.

The mixture was filtered, and washed with ethyl acetate to obtain 7 mg (0.02 mmol) of 1-((8-amino-5,6,7,8-tetrahydronaphthalene-1-yl)methyl)-2-thio-1,2,3,5-tetrahydro-4H-pyrrol o[3,2-d]pyrimidin-4-one hydrochloride (yield: 26.9%). LC-MS: [M+H]⁺=327.2. ¹H NMR (400 MHz, DMSO-d₆) δ 12.52 (s, 2H), 8.21 (s, 3H), 7.36 (d, J=2.7 Hz, 1H), 7.24-7.05 (m, 2H), 6.57 (d, J=7.5 Hz, 1H), 6.14 (d, J=15.5 Hz, 1H), 5.90 (s, 1H), 5.86 (s, 1H), 5.77 (dd, J=6.8 Hz, 16.0 Hz, 2H), 4.89 (s, 1H), 2.84 (m, 2H), 2.24-2.11 (m, 1H), 1.98-1.82 (m, 3H).

### Step I: synthesis of compounds L-1 and L-2

Tert-butyl(tert-butoxycarbonyl)(8-((4-oxo-2-thio-2,3,4,5-tetrahydro-1H-pyrrolo[3,2-d]p yrimidin-1-yl)methyl)-1,2,3,4-tetrahydronaphthalene-1-yl)carbamate (1 g, 1.90 mmol) was fed to SFC for separation (DAICELCHIRALCEL^{®}OZ column (250*25 mm 10 µm), Supercritical CO₂ and MEOH (+0.1% 7.0mol/l Ammonia in MEOH) were used as mobile phases for chiral separation preparation, so as to obtain an isomer L-1 (RT=2.82 min, 400 mg, yield=40%, ee>99%) and L-2 (RT=3.87 min, 450 mg, yield=45%, ee>99%). Preparation of compound 7A:
A compound L-1 (400 mg, 0.76 mmol) was dissolved in dichloromethane (10 mL), a dioxane hydrochloride solution (5 mL) was added to a reaction solution, and the reaction solution was stirred at a room temperature for 2 hours. At the end of the reaction, the reaction solution was concentrated to dryness. Then a sodium bicarbonate aqueous solution was used to adjust a pH of the solution to 8, ethyl acetate (15 mL×2) was used for extraction, and organic phases were combined, dried with anhydrous sodium sulfate, filtered, and subjected to spin drying. The resulting residue was purified using a reversed-phase column (an eluent: 0.1% ammonia water solution), so as to obtain 208.42 mg of compound 7A (yield 84%). LCMS: [M+H]+=327.2; (ee> 99%., RT=3.932 min, column DEA C4 OJ). Preparation of compound 7B:

A compound L-2 (450 mg, 0.85 mmol) was dissolved in dichloromethane (10 mL), a dioxane hydrochloride solution (5 mL) was added to a reaction solution, and the reaction solution was stirred at a room temperature for 2 hours. At the end of the reaction, the reaction solution was concentrated to dryness. Then a sodium bicarbonate aqueous solution was used to adjust a pH of the solution to 8, ethyl acetate (15 mL×2) was used for extraction, and organic phases were combined, dried with anhydrous sodium sulfate, filtered, and subjected to spin drying. The resulting residue was purified using a reversed-phase column (an eluent: 0.1% ammonia water solution), so as to obtain 216.08 mg of compound 7B (yield 77%). LCMS: [M+H]+=327.2; (ee> 99%., RT=3.770 min, column DEA C4 OJ).

### Examples 8-14

Referring to a preparation method of Example 6, compounds 8-14 of the following formulas was prepared respectively.

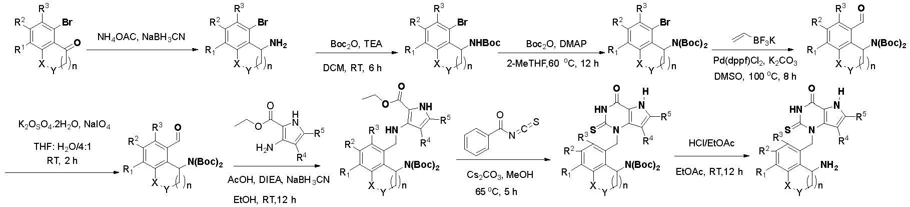

| Compound | Structure | Step A Intermediate | Compound | Structure | Step A Intermediate |
|---|---|---|---|---|---|
| Compound 8 | 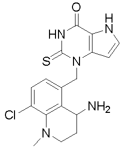 LC-MS: [M+H]⁺=376.1 | 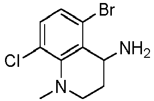 | Compound 12 | 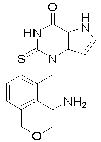 LC-MS: [M+H]⁺=329.1 | 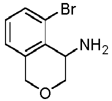 |
| Compound 9 | 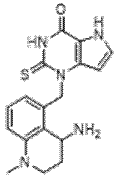 LC-MS: [M+H]⁺=342.1 | 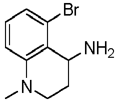 | Compound 13 | 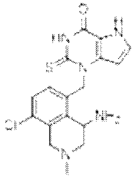 LC-MS: [M+H]⁺=376.1 | 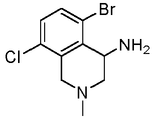 |
| Compound 10 | 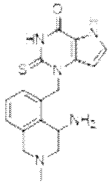 LC-MS: [M+H]⁺=342.1 | 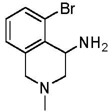 | Compound 14 | 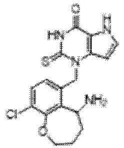 LC-MS: [M+H]⁺=377.2 | 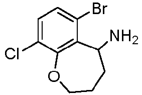 |
| Compound 11 | 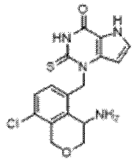 LC-MS: [M+H]⁺=363.2 | 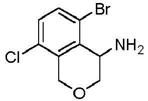 | | | |

### Example 15

### 1-((5-amino-2,3,4,5-tetrahydrobenzo[b]oxypyrimidin-6-yl)methyl)-2-thio-1,2,3,5-tetrah ydroxy-4H-pyrrolo[3,2-d]pyrimidin-4-one, compounds 15A and 15B

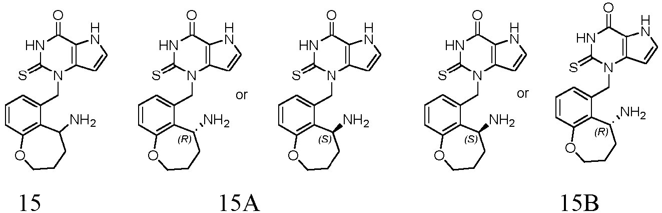

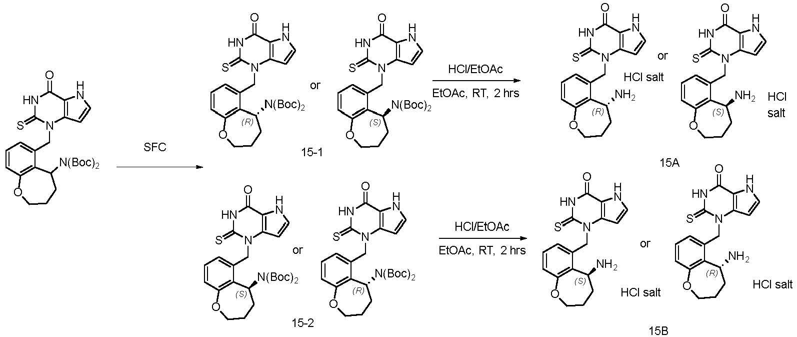

### Step A: synthesis of compounds

### tert-butyl(R)(tert-butoxycarbonyl)(6-((4-oxo-2-thio-2,3,4,5-tetrahydro-1H-pyrrolo[3,2-d]pyrimidin-1-yl)methyl)-2,3,4,5-tetrahydrobenzo[b]oxypyrimidin-5-yl)carbamate and tert-butyl(S)(tert-butoxycarbonyl)(6-((4-oxo-2-thio-2,3,4,5-tetrahydro-1H-pyrrolo[3,2-d]pyrimidin-1-yl)methyl)-2,3,4,5-tetrahydrobenzo[b]oxypyrimidin-5-yl)carbamate

Tert-butyl(R)(tert-butoxycarbonyl)(6-((4-oxo-2-thio-2,3,4,5-tetrahydro-1H-pyrrolo[3,2-d]pyrimidin-1-yl)methyl)-2,3,4,5-tetrahydrobenzo[b]oxypyrimidin-5-yl)carbamate was fed to SFC for separation (DAICELCHIR ALCEL^{®} OJ column (250*25 mm 10µm), Supercritical CO₂ and MEOH (+0.1% 7.0mol/l Ammonia in MEOH) were used as mobile phases for chiral separation preparation, and a separated solvent was subjected to low-temperature spin drying. 120 mg of white solid 15-1, tert-butyl(R or S)(tert-butoxycarbonyl)(6-((4-oxo-2-thio-2,3,4,5-tetrahydro-1H-pyrrolo[3,2-d]pyrimidin-1-y l)methyl)-2,3,4,5-tetrahydrobenzo[b]oxypyrimidin-5-yl)carbamate (RT=1.46 min, ee>99%) and 115 mg of 15-2, tert-butyl(S or R)(tert-butoxycarbonyl)(6-((4-oxo-2-thio-2,3,4,5-tetrahydro-1H-pyrrolo[3,2-d]pyrimidin-1-yl)methyl)-2,3,4,5-tetrahydrobenzo[b]oxypyrimidin-5-yl)carbamate (RT=2.47min, ee>99%) were obtained respectively.

### Step B: synthesis of compound 15A hydrochloride:

Tert-butyl(R)(tert-butoxycarbonyl)(6-((4-oxo-2-thio-2,3,4,5-tetrahydro-1H-pyrrolo[3,2-d]pyrimidin-1-yl)methyl)-2,3,4,5-tetrahydrobenzo[b]oxypyrimidin-5-yl)carbamate (120 mg, 0.22 mmol) was dissolved in ethyl acetate (2 mL), an ethyl acetate hydrochloride solution (4 mL) was added to a reaction solution, and the reaction solution was stirred at a room temperature for 2 hours. The reaction solution was directly subjected to spin drying. Without further purification, 70.95 mg of white solid 15A hydrochloride was obtained by direct freeze-drying.

LCMS: RT=1.233 min, [Ms+H]+=343.2; 1H NMR (400 MHz, DMSO) δ 12.50 (s, 1H), 12.39 (s, 1H), 8.40 (s, 3H), 7.33 (s, 1H), 7.19 (t, J=8.0 Hz, 1H), 6.98 (d, J=8.0 Hz, 1H), 6.42 (d, J=8.0 Hz, 1H), 6.22 (d, J=2.0 Hz, 1H), 6.01 (d, J=16.4Hz, 1H), 5.74 (d, J=16.4 Hz, 1H), 4.89 (s, 1H), 4.46 (d, J=11.2 Hz, 1H), 3.62 (t, J=11.6 Hz, 1H), 2.35-2.20 (m, 2H), 1.87-1.80 (m, 2H).

### Step C: synthesis of compound 15B hydrochloride:

Tert-butyl(S)(tert-butoxycarbonyl)(6-((4-oxo-2-thio-2,3,4,5-tetrahydro-1H-pyrrolo[3,2-d]pyrimidin-1-yl)methyl)-2,3,4,5-tetrahydrobenzo[b]oxypyrimidin-5-yl)carbamate (115 mg, 0.21 mmol) was dissolved in ethyl acetate (2 mL), an ethyl acetate hydrochloride solution (4 mL) was added to a reaction solution, and the reaction solution was stirred at a room temperature for 2 hours. The reaction solution was directly subjected to spin drying. Without further purification, 70.28 mg of pink solid 15B hydrochloride was obtained by direct freeze-drying.

LCMS: RT=1.223 min, [Ms+H]+=343.2; 1H NMR (400 MHz, DMSO) δ 12.50 (s, 1H), 12.38 (s, 1H), 8.42 (s, 3H), 7.33 (t, J=2.8 Hz, 1H), 7.19 (t, J=8.0 Hz, 1H), 6.98 (d, J=8.0 Hz, 1H), 6.41 (d, J=7.6 Hz, 1H), 6.24 (s, 1H), 6.01 (d, J=16.4 Hz, 1H), 5.74 (d, J=16.4 Hz, 1H), 4.88 (s, 1H), 4.46 (d, J=11.2 Hz, 1H), 3.62 (t, J=11.6 Hz, 1H), 2.34-2.25 (m, 2H), 1.81-1.80 (m, 2H).

### Example 16

### 1-(4-aminobenzopyran-5-yl)methyl)-7-methyl-2-thio-1,2,3,5-tetrahydro-4H-pyrrolo[3,2 -d]pyrimidin-4-one

### Synthetic route:

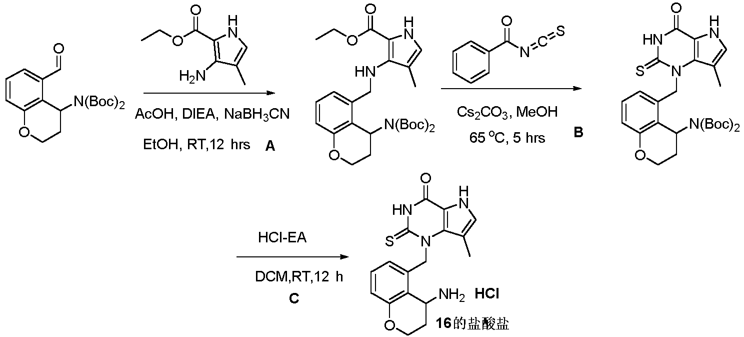

### Step A: synthesis of compound ethyl 3-((4-(bis(tert-butoxycarbonyl)amino)chroman-5-ylmethyl)amino)-4-methylpyrrole-2-c arboxylate

At a room temperature, ethyl 3-amino-4-methylpyrrole-2-carboxylate (80 mg, 0.39 mmol) and DIEA (0.07 mL, 0.39 mmol) were added to ethanol (10 mL) and allowed to react for 5 minutes, glacial acetic acid (0.02 mL, 0.39 mmol) was added, and a mixture reacted at a room temperature for 30 minutes;
tert-butyl(tert-butoxycarbonyl)(5-formylchroman-4-yl)carbamate (124 mg, 0.33 mmol) was added, and a mixture reacted at a room temperature for 1 hour; and sodium cyanoborohydride (62 mg, 0.99 mmol) was added, and a mixture reacted at a room temperature for 12 hours. At the end of the reaction, ethanol in a reaction solution was subjected to spin drying, 20 mL of water was added, ethyl acetate (20 mL×3) was used for extraction, and organic phases were combined, dried with anhydrous sodium sulfate, filtered, and concentrated to dryness. The resulting residue was purified by reverse phase column chromatography (an eluent: 0.1% ammonium bicarbonate aqueous solution:acetonitrile=65%) to obtain 90 mg of white solid product ethyl 3-((4-(bis(tert-butoxycarbonyl)amino)chroman-5-ylmethyl)amino)-4-methylpyrrole-2-carbo xylate (yield: 51.72%).
LC-MS: [M+H]+=529.63; 1H NMR (400 MHz, DMSO) δ 10.60 (s, 1H), 7.17 (t, J=7.9 Hz, 1H), 6.95 (d, J=7.5 Hz, 1H), 6.71 (d, J=8.2 Hz, 1H), 6.58 (d, J=3.1 Hz, 1H), 5.46-5.28 (m, 2H), 4.47-4.32 (m, 2H), 4.17 (dt, J=10.6, 9.8 Hz, 4H), 2.12-2.02 (m, 2H), 2.01 (d, J=11.0 Hz, 3H), 1.23 (dd, J=13.8, 6.5 Hz, 21H).

### Step B: synthesis of compound tert-butyl(5-((7-methyl-4-oxo-2-thio-2,3,4,5-tetrahydro-1H-pyrrolo[3,2-d]pyrimidin-1-y l)methyl)chroman-4-yl)carbamate

At a room temperature, ethyl 3-((4-(bis(tert-butoxycarbonyl)amino)chroman-5-ylmethyl)amino)-4-methylpyrrole-2-carbo xylate (90 mg, 0.17 mmol) and benzoyl isothiocyanate (0.04 mL, 0.2 mmol) ware added to 10 mL of anhydrous methanol, a mixture reacted at a room temperature for 2 hours, then cesium carbonate (222 mg, 0.68 mmol) was added, and a mixture reacted at 65°C for 2 hours. At the end of the reaction, methanol in a reaction solution was subjected to spin drying, 20 mL of water was added, ethyl acetate (30 mL×3) was used for extraction, and organic phases were combined, dried with anhydrous sodium sulfate, filtered, and concentrated to dryness. The resulting residue was purified by reverse phase column chromatography (an eluent: 0.1% ammonium bicarbonate aqueous solution:acetonitrile=65%) to obtain 90 mg of white solid product tert-butyl(5-((7-methyl-4-oxo-2-thio-2,3,4,5-tetrahydro-1H-pyrrolo[3,2-d]pyrimidin-1-yl)me thyl)chroman-4-yl)carbamate (yield: 97.61%).

LC-MS: [M+H]+=542.65; 1H NMR (400 MHz, DMSO) δ 12.31 (s, 2H), 7.10 (dd, J=33.8, 25.9 Hz, 2H), 6.73 (d, J=8.1 Hz, 1H), 6.26 (s, 1H),6.03-4.80 (m, 3H), 4.41 (s, 1H), 4.19-4.03 (m, 1H), 2.24 (d, J=22.5 Hz, 2H), 1.99 (dd, J=45.5, 37.5 Hz, 3H),1.28 (d, J=32.4 Hz, 18H).

### Step C: synthesis of compound

### 1-(4-aminobenzopyran-5-yl)methyl)-7-methyl-2-thio-1,2,3,5-tetrahydro-4H-pyrrolo[3,2 -d]pyrimidin-4-one hydrochloride

At a room temperature,
tert-butyl(5-((7-methyl-4-oxo-2-thio-2,3,4,5-tetrahydro-1H-pyrrolo[3,2-d]pyrimidin-1-yl)me thyl)chroman-4-yl)carbamate (90 mg, 0.17 mmol) was dissolved in DCM (10 mL), hydrochloric acid-ethyl acetate (1.3 mL, 5 mmol) was added, and a mixture reacted at a room temperature for 12 hours. At the end of the reaction, dichloromethane was subjected to spin drying. The resulting residue was added with dichloromethane (30 mL), beaten for 1 hour and filtered to obtain 40 mg of solid product 1-(4-aminobenzopyran-5-yl)methyl)-7-methyl-2-thio-1,2,3,5-tetrahydro-4H-pyrrolo[3,2-d]p yrimidin-4-one hydrochloride (yield: 70.5%).
LC-MS: [M+H]+=342.42; 1H NMR (400 MHz, DMSO) δ 12.33 (d, J=28.3 Hz, 2H), 8.62 (s, 3H), 7.20 (t, J=8.0 Hz, 2H), 6.80 (d, J=8.2 Hz, 1H), 6.33 (d, J=7.5 Hz, 1H), 4.87 (s, 1H), 4.40 (d, J=7.9 Hz, 1H), 4.28 (t, J=12.1 Hz, 1H),2.36 (t, J=14.3 Hz, 1H), 2.14 (dd, J=17.1, 7.0 Hz, 1H).

### Example 17

### 1-((4-aminochroman-5-yl)methyl)-7-methoxyl-2-thio-1,2,3,5-tetrahydro-4H-pyrrolo[3,2 -d]pyrimidin-4-one

### Synthetic route:

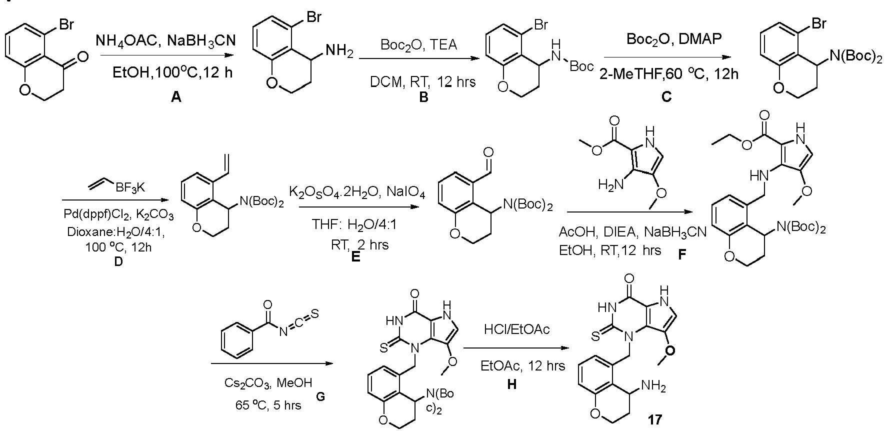

### Step A: 5-bromo-4-benzodihydropyran-amine

At a room temperature, 5-bromo-4-benzodihydropyranone (45 g, 198 mmol) was dissolved in anhydrous ethanol (500 mL), ammonium acetate (124 g, 1.98 mmol) and sodium cyanoborohydride (76 g, 0.99 mmol) were added, and a mixture was refluxed at 100°C for 12 hours. After LC-MS showed that the reaction was completed, a solvent in the solution was subjected to spin drying, water (300 mL) was added, a saturated sodium bicarbonate solution was used to adjust a pH to 10, ethyl acetate (500 mL×2) was used for extraction, and organic phases were combined, dried with anhydrous sodium sulfate, filtered, and concentrated. The crude product was purified by silica gel column chromatography (an eluent: dichloromethane/methanol=20/1) to obtain 29 g of white solid **5-bromo-4-benzodihydropyran-amine** (yield: 64%).
¹H NMR (400 MHz, DMSO) δ 7.16-7.01 (m, 2H), 6.86-6.76 (m, 1H), 4.32-4.17 (m, 2H), 4.09-3.98 (m, 1H), 1.96-1.77 (m, 2H).

### Step B: synthesis of compound tert-butyl(5-bromo-4-benzodihydropyran-amine)carbamate

**5-bromo-4-benzodihydropyran-amine** (29 g, 127 mmol), di-tert-butyl dicarbonate (56 g, 254 mmol), and triethylamine (39 g, 381 mmol) were dissolved in dichloromethane (300 mL), and a mixture reacted at a room temperature for 12 hours. At the end of the reaction, 200 mL of water was added to a reaction solution, dichloromethane (300 mL×3) was used for extraction, and organic phases were combined, washed with a NaHCO3 aqueous solution, dried with anhydrous sodium sulfate, filtered, and subjected to spin drying. The resulting residue was purified by silica gel column chromatography (an eluent: petroleum ether/ethyl acetate=10/1) to obtain 30 g of tert-butyl(5-bromo-4-benzodihydropyran-amine)carbamate (yield: 71%).
¹H NMR (400 MHz, DMSO) δ 7.41 (d, J=7.6 Hz, 1H), 7.16-7.09 (m, 2H), 6.81 (dd, J=8.0, 1.2 Hz, 1H), 4.63 (s, 1H), 4.25 (d, J=10.8 Hz, 1H), 4.04 (dd, J=17.2, 6.4 Hz, 1H), 1.98-1.78 (m, 2H), 1.42 (s, 9H).

### Step C: synthesis of compound tert-butyl(5-bromo-4-benzodihydropyran)(tert-butoxycarbonyl)carbamate

Tert-butyl(5-bromo-4-benzodihydropyran-amine)carbamate (30 g, 91.4 mmol), di-tert-butyl dicarbonate (199 g, 914 mmol), and 4-dimethylaminopyridine (22 g, 183 mmol) were dissolved in dimethyltetrahydrofuran (300 mL), and a mixture reacted at 60°C for 12 hours. At the end of the reaction, the mixture was cooled to a room temperature and subjected to spin drying, 100 mL of water was added, ethyl acetate (300 mL×3) was used for extraction, and organic phases were combined, washed with a NaHCO3 aqueous solution, dried with anhydrous sodium sulfate, filtered, and subjected to spin drying. The resulting residue was purified by silica gel column chromatography (an eluent: petroleum ether/ethyl acetate=10/1) to obtain 32 g of yellow solid tert-butyl(5-bromo-4-benzodihydropyran)(tert-butoxycarbonyl)carbamate (yield: 82%). ¹H NMR (400 MHz, DMSO) δ 7.19-7.08 (m, 2H), 6.87-6.78 (m, 1H), 5.26 (t, J=4.2 Hz, 1H), 4.37-4.32 (m, 1H), 4.27-4.15 (m, 1H), 2.16-2.00 (m, 2H), 1.34 (s, 18H).

### Step D: synthesis of compound

### tert-butyl(5-vinyl-4-benzodihydropyran)(tert-butoxycarbonyl)carbamate

Tert-butyl(5-bromo-4-benzodihydropyran)(tert-butoxycarbonyl)carbamate (31 g, 72 mmol), potassium vinyltrifluoroborate (12.5 g, 93.6 mmol),
1,1'-bis(diphenylphosphine)ferrocene palladium chloride (5.2 g, 7.2 mmol), and potassium carbonate (2.9 g, 216 mmol) were dissolved in anhydrous 1,4-dioxane (300 mL) and water (75 mL), and a mixture reacted at 100°C under nitrogen protection for 12 hours. At the end of the reaction, the mixture was cooled to a room temperature, a reaction solution was filtered through diatomaceous earth, filtrate was collected, then 100 mL of water was added, ethyl acetate (300 mL×3) was used for extraction, and organic phases were combined, washed with a NaCl aqueous solution, dried with anhydrous sodium sulfate, filtered, and subjected to spin drying. The resulting residue was purified by silica gel column chromatography (an eluent: petroleum ether/ethyl acetate=10/1) to obtain 16 g of yellow solid tert-butyl(5-vinyl-4-benzodihydropyran)(tert-butoxycarbonyl)carbamate (yield: 59%).
¹H NMR (400 MHz, DMSO) δ 7.15-7.06 (m, 1H), 7.06 (d, J=7.2 Hz, 1H), 6.76-6.69 (m, 2H), 5.65 (dd, J=17.2, 1.2 Hz, 1H), 5.41 (t, J=5.2 Hz, 1H), 5.30-5.21 (m, 1H), 4.36 (dd, J=7.2, 3.6 Hz, 1H), 4.17-4.08 (m, 1H), 2.17-2.02 (m, 3H), 1.26 (s, 18H).

### Step E: synthesis of tert-butyl(5-formyl-4-benzodihydropyran)(tert-butoxycarbonyl)carbamate

Tert-butyl(5-vinyl-4-benzodihydropyran)(tert-butoxycarbonyl)carbamate (5 g, 13.3 mmol) was dissolved in a mixed solution of tetrahydrofuran (120 mL) and water (30 mL), potassium osmate (490 mg, 1.33 mmol) was added to a reaction solution, the reaction solution was stirred at a room temperature for 30 minutes, then sodium periodate (11.4 g, 53.2 mmol) was added to the reaction solution, and the reaction solution reacted at a room temperature for 2 hours. At the end of the reaction, 50 mL of water was added to the reaction solution, ethyl acetate (200 mL×2) was used for extraction, and organic phases were combined, dried with anhydrous sodium sulfate, filtered, and subjected to spin drying. The resulting residue was purified by silica gel column chromatography (an eluent: petroleum ether/ethyl acetate=10/1) to obtain 2.6 g of yellow solid tert-butyl(5-formyl-4-benzodihydropyran)(tert-butoxycarbonyl)carbamate (yield: 52%). LCMS: m/z (ESI), [M+Na]⁺=400.1. ¹H NMR (400 MHz, DMSO) δ 10.02 (s, 1H), 7.42-7.39 (m, 2H), 7.14-7.01 (m, 1H), 5.89 (t, J=5.6 Hz, 1H), 4.43-4.37 (m, 1H), 4.23-4.18 (m, 1H), 2.2-2.12 (m, 2H), 1.26 (s, 18H).

### Step F: synthesis of compound ethyl 3-(((4-(bis(tert-butoxycarbonyl)amino)pyran-5-yl)methyl)amino)-4-methoxyl-1H-pyrro le-2-carboxylate

Ethyl 3-amino-4-methoxy-1H-pyrrole-2-carboxylate (118 mg, 0.64 mmol) was dissolved in 15 mL of anhydrous ethanol, then N,N-diisopropylethylamine (83 mg, 0.64 mmol) and glacial acetic acid (38 mg, 0.64 mmol) were added to a mixture, the mixture was stirred at a room temperature for 10 minutes, then tert-butyl(5-formyl-4-benzodihydropyran)(tert-butoxycarbonyl)carbamate (200 mg, 0.53 mmol) was added to the mixture, and stirred at a room temperature for 2 hours, then sodium cyanoborohydride (100 mg, 1.6 mmol) was added to the mixture, and the mixture reacted at a room temperature for 3 hours. At the end of the reaction, the anhydrous ethanol in the reaction solution was subjected to spin drying. 20 mL of water and ethyl acetate (30 mL×3) were used for extraction, and organic phases were combined, dried with anhydrous sodium sulfate, filtered, and subjected to spin drying. The resulting residue was purified using a reversed-phase column (an eluent: 0.1% ammonium bicarbonate solution) to obtain 150 mg of yellow solid **ethyl 3-(((4-(bis(tert-butoxycarbonyl)amino)pyran-5-yl)methyl)amino)-4-methoxyl-1H-pyrro le-2-carboxylate** (yield: 52%). LCMS: RT=1.99 min, [Ms+H]+=546; ¹H NMR (400 MHz, DMSO) δ 10.34 (s, 1H), 7.11 (t, *J*=7.9 Hz, 1H), 6.88 (d, *J*=7.5 Hz, 1H), 6.65 (d, *J*=8.2 Hz, 1H), 6.54 (d, *J*=3.5 Hz, 1H), 5.41 (s, 1H), 5.31 (d, *J*=6.4 Hz, 1H), 4.45 (ddd, *J*=18.0*,* 13.0, 7.4 Hz, 3H), 4.29-4.09 (m, 5H), 3.55 (s, 3H), 2.17-2.01 (m, 3H), 1.25 (d, *J*=10.1 Hz, 18H).

### Step G: synthesis of compound tert-butyl(tert-butoxycarbonyl)(5-((7-methoxyl-4-oxo-2-thio-2,3,4,5-tetrahydro-1H-pyr rolo[3,2-d]pyrimidin-1-yl)methyl)pyran-4-yl)carbamate

Ethyl 3-(((4-(bis(tert-butoxycarbonyl)amino)pyran-5-yl)methyl)amino)-4-methoxyl-1H-pyrrole-2-carboxylate (150 mg, 0.27 mmol) and benzoyl isothiocyanate (68 mg, 0.42 mmol) ware dissolved in methanol (15 mL), a mixture was stirred at a room temperature for 3 hours, then cesium carbonate (274 mg, 0.84 mmol) was added, and a reaction solution reacted at 65°C for 3 hours. At the end of the reaction, the anhydrous methanol in the reaction solution was subjected to spin drying. The resulting residue was purified using a reversed-phase column (an eluent: 0.1% ammonium bicarbonate solution) to obtain 150 mg of white solid tert-butyl(tert-butoxycarbonyl)(5-((7-methoxyl-4-oxo-2-thio-2,3,4,5-tetrahydro-1H-pyrrolo[ 3,2-d]pyrimidin-1-yl)methyl)pyran-4-yl)carbamate (yield: 76%). LCMS: RT=1.82min, [Ms+H]+=559.

### Step H: synthesis of compound 1-((4-aminochroman-5-yl)methyl)-7-methoxyl-2-thio-1,2,3,5-tetrahydro-4H-pyrrolo[3,2 -d]pyrimidin-4-one hydrochloride

Tert-butyl(tert-butoxycarbonyl)(5-((7-methoxyl-4-oxo-2-thio-2,3,4,5-tetrahydro-1H-py rrolo[3,2-d]pyrimidin-1-yl)methyl)pyran-4-yl)carbamate (150 mg, 0.27 mmol) was dissolved in EA (2 mL), an ethyl acetate hydrochloride solution (2 mL, 8.1 mmol) was added to a reaction solution, and the reaction solution was stirred at a room temperature for 2 hours. The reaction solution was filtered to obtain a crude white solid. The resulting crude filter cake was freeze-dried to obtain 35 mg of white solid product 1-((4-aminochroman-5-yl)methyl)-7-methoxyl-2-thio-1,2,3,5-tetrahydro-4H-pyrrolo[3,2-d]p yrimidin-4-one hydrochloride. LCMS: RT=1.29 min, [Ms+H]+=359; 1H NMR (400 MHz, DMSO) δ 12.35 (s, 1H), 12.14 (s, 1H), 8.59 (s, 3H), 7.25-7.10 (m, 2H), 6.76 (d, J=8.2 Hz, 1H), 6.43 (d, J=7.7 Hz, 1H), 6.31-5.95 (m, 1H), 5.55 (s, 1H), 4.87 (s, 1H), 4.37 (dt, J=20.6, 10.4 Hz, 2H), 3.60 (d, J=29.0 Hz, 3H), 2.43 (d, J=14.8 Hz, 1H), 2.13 (t, J=13.8 Hz, 1H).

### Example 18: Evaluation Experiment on Inhibition of Human Myeloperoxidase (hMPO) Activity by Compound of the Present Disclosure in Vitro

50 µL of 2×substrate (composed of 3'-(p-aminophenyl) fluorescein and H₂O₂, with 3'-(p-aminophenyl) fluorescein and H₂O₂ both at Km concentration, purchased from Invitrogen, A36003) to a black opaque low-protein-adsorption 96-well plate (purchased from PerkinElmer, 6005270), and 45 µL of 2×compound at different concentrations (DMSO final concentration of 1%) was added. After thorough blowing and uniform mixing of a mixture, 5 µL of 2 µg/mL human myeloperoxidase (purchased from PLANTA NATURAL, 700-03-001) was added and mixed uniformly, and an enzyme-linked immunosorbent assay reader was immediately used at a wavelength of Ex/Em:488/520nm to read fluorescence values for 0-30 minutes (read every 20 seconds) using a dynamic mode. Different concentrations of compound inhibitors were calculated: inhibition rate (%)=[1-(Slope_{Control} - Slope_{cpd} )/ Slope_{Control} -Slope_{Blank} )]* 100, where Slope_{Blank} is a slope of a control without the compound and an MPO well at 0-5 minutes (Ex/Em: 544/620nm reading/reaction time t); SlopeControl is a slope of the well without the compound (containing 1% DMSO) at 0-5 minutes (linear reaction stage); and Slope_{cpd} is a slope of the compound at 0-5 minutes. Using a logarithmic value of the compound concentration as an abscissa and the inhibition rate as an ordinate, a 4-parameter nonlinear regression curve was fitted to calculate the IC₅₀ value (Y=Bottom+(Top-Bottom)/(1+10^((LogIC₅₀-X)*HillSlope)), where Hillslope represents the slope of the fitted curve, and IC₅₀ represents the half inhibitory concentration. Test results were shown in Table 1.

**Table 1 Inhibitory activity of compound on human myeloperoxidase (hMPO) peroxidation cycle in vitro**

| **Example compound** | **IC₅₀ (nM)** |
|---|---|
| 1 | 51.57 |
| 2 | 33.04 |
| 3 | 30.96 |
| 5 | 16.14 |
| 6 | 8.02 |
| 6A | 4.64 |
| 7 | 10.66 |
| 7A | 4.83 |
| 15A | 9.92 |
| 16 | 19.55 |
| 17 | 23.43 |

Conclusion: The compound of the present disclosure has the effect of inhibiting hMPO activity.

### Example 19: Pharmacokinetic Study of Compound of the Present Disclosure in Rats

### 19.1 Experimental material

SD rats: male, 180-350g, purchased from Zhejiang Vital River Laboratory Animal Technology Co., Ltd.

Reagents: dimethyl sulfoxide (DMSO), polyethylene glycol 400 (PEG-400), normal saline, acetonitrile, formic acid, propranolol (internal standard) are all commercially available.

Instrument: Thermo Fisher Scientific LC-MS (Ultimate 3000 UPLC, TSQ QUANTUMN ULTRA triple quadrupole mass spectrometer).

### 19.2 Experimental method

The compound was weighed and dissolved in a DMSO-PEG-400 normal saline (5:60:35, v/v/v) system. After intravenous administration or intragastric administration to rats, 200 µL of venous blood was collected in an EDTA-K2 anticoagulation tube at 15 minutes, 30 minutes, 1 hour, 2 hours, 5 hours, 7 hours, and 24 hours (additional 5 minutes for an IV group) and centrifuged at 12000 rpm for 2 minutes. Plasma was taken and frozen and preserved at -80°C for testing. A certain amount of samples for test was precisely weighed and dissolved in DMSO to 2 mg/mL as a stock solution. An appropriate amount of compound stock solution was accurately sucked, and diluted by adding acetonitrile to prepare a standard series solution. 4 µL of each of the above standard series solution was accurately sucked, and added to 36 µL of blank plasma, vortex mixing was performed to prepare plasma samples equivalent to plasma concentrations of 1 ng/mL, 3 ng/mL, 5 ng/mL, 10 ng/mL, 30 ng/mL, 100 ng/mL, 300 ng/mL, 1000 ng/mL, and 3000 ng/mL. Dual sample analysis was performed for each concentration to establish a standard curve. 30 µL of plasma (5-fold dilution of plasma of intravenous administration for 5 minutes, 15 minutes, and 30 minutes) was taken, a propranolol acetonitrile solution (internal standard, 50 ng/mL) was added to precipitate the protein, after 100 µL water was added for even vortex mixing, centrifugation was performed at 4000 rpm for 5 minutes, and supernatants were taken for LC-MS/MS analysis. LC-MS detection conditions were as follows:
chromatographic column: YMC-Triart C₁₈ 50×3.0mmI.D. S-3µm,12nm

A mobile phase A: water (0.1% formic acid), a mobile phase B: acetonitrile, a flow rate: 0.5 mL/min, gradient elution:

| Time (min) | A(%) | B(%) |
|---|---|---|
| 0 | 90% | 10% |
| 0.60 | 90% | 10% |
| 1.50 | 10% | 90% |
| 2.50 | 10% | 90% |
| 2.51 | 90% | 10% |
| 3.30 | 90% | 10% |

### 19.3 Data processing

After LC-MS detection of blood drug concentration, WinNonlin 6.1 software was used to calculate pharmacokinetic parameters using non compartmental model method. The test results were shown in Table 2.

**Table 2 Pharmacokinetic results of the compound of the present disclosure in rats**

| Example compound | Dosage (mg/kg) Administration route | Cmax (ng/ml) | AUClast (h*ng/ml) | T_{1/2}(h) | Cl (ml/min/k g) | F(%) |
|---|---|---|---|---|---|---|
| 6 | 1, i.v | 620 | 827 | 3.36 | 19.9 | 48.6 |
| | 5, p.o | 305 | 2010 | 5.13 | | |
| 6A | 1, i.v | 1.9 | 1270 | 4.98 | 13 | 57.2 |
| | 5, p.o | 1.7 | 3630 | 3.66 | | |
| 7A | 1, i.v | 0.8 | 293 | 2.59 | 52.7 | 83.3 |
| | 5, p.o | 0.5 | 1220 | 3.44 | | |
| Example 3 (CN201580065064.2) | 1, i.v | 394 | 1190 | 3.87 | 13.9 | 25.6 |
| | 5, p.o | 105 | 1520 | 5.26 | | |

According to the experimental results in Table 2, it can be seen that the compound of the present disclosure has slower metabolism in rats, higher oral exposure level and bioavailability.

### Example 20: Study on Enzyme Induction Test of Compound of the Present Disclosure

### 20.1 Experimental material and instrument

Material: Williams' Medium E (without phenol red) was purchased from Sigma Aldrich Trading Co., Ltd; Williams' Medium E, Recombinant human insulin, GlutaMAX, and HEPES were purchased from Life Technologies; Isotonic Percoll was purchased from General Electric; Fetal bovine serum was purchased from Corning; Dexamethasone was commercially available; CellTiter-Fluor^{™} Cell Viability Assay kit was purchased from Promega; Taqman Gene Expression Assay probe (20×, CYP1A2, FAM labeled), Taqman Gene Expression Assay probe (20×, CYP2B6, FAM labeled), Taqman Gene Expression Assay probe (20×, CYP3A4, FAM labeled), and Taqman Gene Expression Assay probe (20×, ACTB, VIC labeled) were purchased from AB (Applied Biosystems); and human primary hepatocytes were purchased from BioIVT.

Instruments: QuantStudio 6.

### 20.2 Experimental design

The test compound, a positive inducer, a negative inducer, and internal standard were accurately weighed to prepare a stock solution with the corresponding concentration. A cell thawing solution and incubation buffer were prepared, and related reagents and consumables of mRNA extraction, reverse transcription, and fluorescence quantitative PCR were prepared.

The frozen human primary hepatocytes were thawed and inoculated onto a suitable cell culture plate at a certain density for monolayer cell culture to meet cell growth requirements.

A preheated solution (10 µM) freshly prepared with the incubation buffer and containing the positive/negative inducer or a test substance was added to the corresponding wells, with two replicates at each concentration. Incubation was performed continuously for 3 days, the solution was changed daily, and then an mRNA level of CYP enzyme was tested. The mRNA extraction, reverse transcription, and fluorescence quantitative PCR were experimented according to the instructions of the relevant reagent kit.

### 20.3. Experimental result

The experimental results of the induction effect of the test compound on the mRNA level of the CYP enzyme were shown in Table 3 below.

**Table 3 Experimental result**

| **CYP enzyme** | | **mRNA** |
|---|---|---|
| | **Compound number** | **Relative positive induction percentage (%)** |
| | Example 3 (CN201580065064.2) | 0 |
| CYP1A2 | 6A | -0.29 |
| | 7A | 0.15 |
| | Example 3 (CN201580065064.2) | 27.47 |
| CYP2B6 | 6A | 1.68 |
| | 7A | 8.41 |
| | Example 3 (CN201580065064.2) | 32.89 |
| CYP3A4 | 6A | 2.10 |
| | 7A | 7.93 |

According to the experimental results in Table 3, it can be seen that the compound of the present disclosure has a reduced induction risk compared to the compound of Example 3 (CN201580065064.2).

### Example 21: Study on Enzyme Inhibition Test of Compound of the Present

### Disclosure

### 21.1 Experimental material and instrument

Material: midazolam and testosterone were purchased from Cerilliant and PANPHY; ketoconazole was purchased from MCE; and human liver microsomes were purchased from Corning.

Instruments: AB Sciex5500+.

### 21.2 Experimental design

The inhibitory potential of the compound on cytochrome P450 enzyme CYP3A was evaluated using a phosphate buffer saline (100 mM, pH 7.4) system containing 0.2 mg/mL human liver microsomes. The test concentrations of the compound were 0.068 µM, 0.206 µM, 0.62 µM, 1.85 µM, 5.56 µM, 16.67 µM, and 50 µM. The positive substrates in the incubation system were 1 µM midazolam and 40 µM testosterone (CYP3A), respectively. The concentration of the positive inhibitor ketoconazole (CYP3A) in the incubation system was 0.05 µM. Incubation was carried out at 37°C, and an NADPH solution with a final concentration of 1 mM was added to initiate a reaction. The incubation time was 10 minutes. After incubation, all samples were added to 300 µL of acetonitrile solution containing 3% formic acid and internal standard (0.5 µM tolbutamide) to precipitate the protein, and the metabolic product generation of the labeled substrate was detected by UPLC-MS/MS to calculate an IC₅₀ value of compound inhibition.

### 21.3. Experimental result

The IC₅₀ value of the inhibition effect of the test compound on CYP3A is shown in the table below.

**Table 4 Experimental result**

| **CYP enzyme** | **IC₅₀ (µM)** | | |
|---|---|---|---|
| | Example 3 (CN201580065064.2) | **6A** | **7A** |
| CYP3A (Midazolam) | 7.17 | >50 | >50 |
| CYP3A(Testosterone) | 0.85 | 27.32 | 19.73 |

According to the experimental results in Table 4, it can be seen that the compound of the present disclosure has a reduced risk on CYP3A inhibition compared to the compound of Example 3 (CN201580065064.2).

The above examples are preferred implementations of the present disclosure, but the implementations of the present disclosure are not limited by the above examples. Any other change, modification, substitution, combination and simplification without departing from the spiritual essence and principle of the present invention shall be equivalent replacement methods, which are contained in the scope of protection of the present disclosure.

## Claims

1. A compound of formula (I): 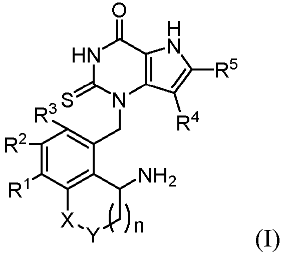 or a stereoisomer, a tautomer or a pharmaceutically acceptable salt thereof, wherein:
R¹, R², R³, R⁴ and R⁵ are independently selected from hydrogen, halogen, hydroxyl, amino, cyano, C₁₋₆ alkyl, C₁₋₆ alkoxyl, alkoxyalkyl, cycloalkyl, aryl or heteroaryl, wherein aryl and heteroaryl may be optionally substituted by one or more groups independently selected from halogen, hydroxyl, cyano, carboxyl, C₁₋₆ alkyl, C₁₋₆ alkoxyl, and halogenated C₁₋₆ alkyl;
X and Y are independently selected from O, CH₂ and NR⁶, and R⁶ is independently selected from hydrogen, halogen, C₁₋₆ alkyl, C₂₋₆ alkenyl, C₁₋₆ alkoxyl, alkoxyalkyl, cycloalkyl, hydroxyl, amino, cyano, cyano-substituted C₁₋₆ alkyl, amino-substituted C₁₋₆ alkyl, halogenated C₁₋₆ alkyl, halogenated C₁₋₆ alkoxyl, -(CH₂)ₙ-C(=O)-R⁷, -(CH₂)ₙ-C(= O)OR⁸, and -(CH₂)ₙ-NHC(=O)-R⁹; R⁷, R⁸ and R⁹ are independently selected from hydrogen and C₁₋₆ alkyl; and
n is 0, 1, 2 or 3.

2. The compound, or the stereoisomer, the tautomer or the pharmaceutically acceptable salt thereof according to claim 1, wherein the compound is represented by formula (II): 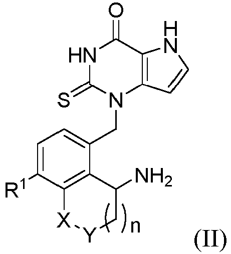 wherein, X, Y, R¹, and n are defined as above.

3. The compound, or the stereoisomer, the tautomer or the pharmaceutically acceptable salt thereof according to claim 1, wherein the C₁₋₆ alkyl is selected from methyl, ethyl, propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl; and
the C₂₋₆ alkenyl is selected from vinyl, 1-propenyl, 2-propenyl, 1-methylvinyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, and 1-ethyl-2-methyl-2-propenyl.

4. The compound, or the stereoisomer, the tautomer or the pharmaceutically acceptable salt thereof according to claim 1, wherein the C₁₋₆ alkoxyl is selected from methoxy, ethoxy, propoxy, isopropoxy, n-butoxy, isobutoxy, sec-butoxy, tert-butoxy, n-pentyloxy, sec-pentyloxy, 1-ethylpropoxy, 2-methylbutoxy, tert-pentyloxy, 1,2-dimethylpropoxy, isopentyloxy, neopentyloxy, n-hexyloxy, isohexyloxy, sec-hexyloxy, tert-hexyloxy, neohexyloxy, 2-methylpentyloxy, 1,2-dimethylbutoxy, and 1-ethylbutoxy; and the alkoxyalkyl is selected from C₁₋₄ alkoxyl C₁₋₄ alkyl, and further selected from methoxymethyl, methoxyethyl, methoxypropyl, methoxybutyl, ethoxymethyl, ethoxyethyl, ethoxypropyl, ethoxybutyl, propoxymethyl, propoxyethyl, propoxypropyl, propoxybutyl, butoxymethyl, butoxyethyl, butoxypropyl, butoxybutyl, etc.

5. The compound, or the stereoisomer, the tautomer or the pharmaceutically acceptable salt thereof according to claim 1, wherein the halogen is selected from fluorine, chlorine, bromine, and iodine, halogenated C₁₋₆ alkyl refers to substitution of one or more hydrogen atom of C₁₋₆ alkyl by halogen, halogenated C₁₋₆ alkoxyl refers to substitution of one or more hydrogen atom of C₁₋₆ alkoxyl by halogen, cyano-substituted C₁₋₆ alkyl refers to substitution of one or more hydrogen atom of C₁₋₆ alkyl by cyano, and amino-substituted C₁₋₆ alkyl refers to substitution of one or more hydrogen atom of C₁₋₆ alkyl by amino.

6. The compound, or the stereoisomer, the tautomer or the pharmaceutically acceptable salt thereof according to claim 1, wherein the aryl is selected from phenyl; and the heteroaryl is selected from 5- to 12- membered heteroaryl, and the 5- to 12- membered heteroaryl is selected from

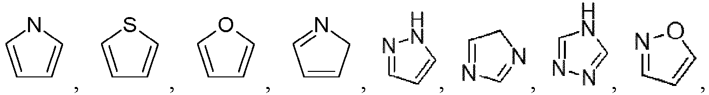

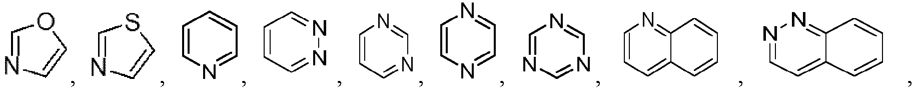

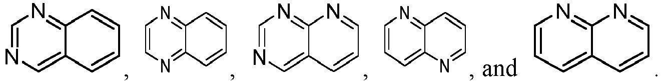

7. The compound, or the stereoisomer, the tautomer or the pharmaceutically acceptable salt thereof according to claim 1, wherein the cycloalkyl is selected from C₃₋₆ cycloalkyl, and the C₃₋₆ cycloalkyl is selected from cyclopropyl, cyclobutyl, cyclopentyl, and cyclohexyl.

8. The compound, or the stereoisomer, the tautomer or the pharmaceutically acceptable salt thereof according to claim 1, wherein R¹ is selected from hydrogen or chlorine;
R², R³ and R⁵ are selected from hydrogen;
R⁴ is selected from hydrogen, methyl, and methoxy;
X is O, CH₂, N-CH₃, or N-C(=O)-O-CH₂-CH₃;
Y is O, CH₂, or N-CH₃; and
N is 0, 1 or 2.

9. The compound, or the stereoisomer, the tautomer or the pharmaceutically acceptable salt thereof according to claim 1, being selected from the following compounds:
| | Structural formula | | Structural formula | | Structural formula |
|---|---|---|---|---|---|
| 1 | 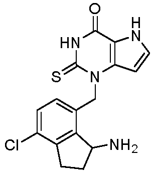 | 2 | 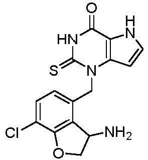 | 3 | 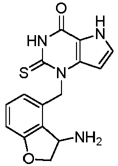 |
| 4 | 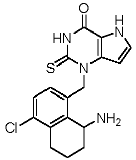 | 5 | 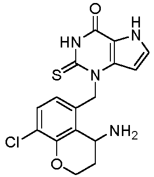 | 6 | 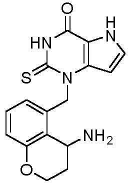 |
| 7 | 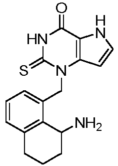 | 8 | 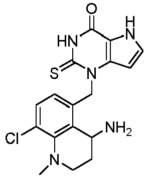 | 9 | 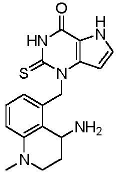 |
| 10 | 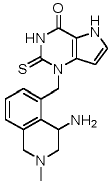 | 11 | 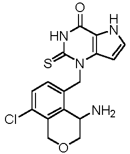 | 12 | 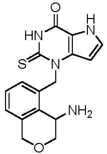 |
| 13 | 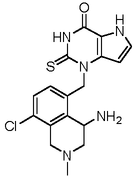 | 14 | 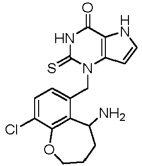 | 15 | 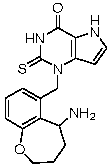 |
| 16 | 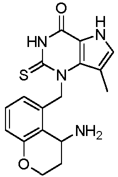 | 17 | 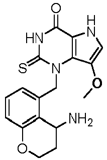 | | |

10. The compound, or the stereoisomer, the tautomer or the pharmaceutically acceptable salt thereof according to claim 1, being selected from the following compounds:
| | Structural formula | | Structural formula |
|---|---|---|---|
| 1A | 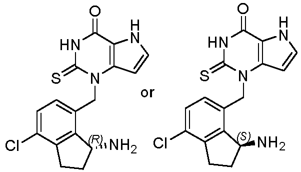 | 1B | 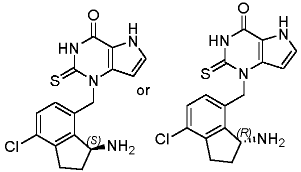 |
| 2 | 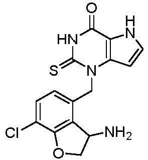 | 3 | 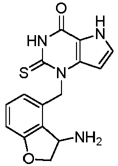 |
| 4 | 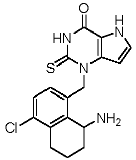 | 5 | 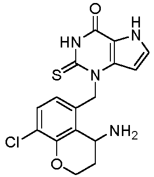 |
| 6A | 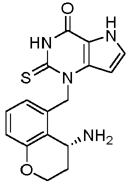 | 6B | 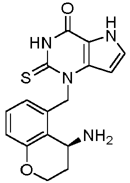 |
| 7A | 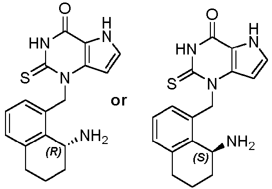 | 7B | 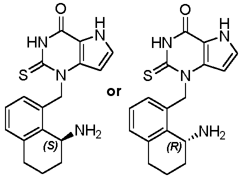 |
| 8 | 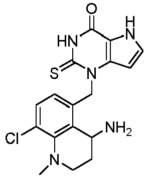 | 9 | 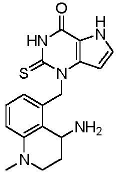 |
| 10 | 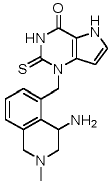 | 11 | 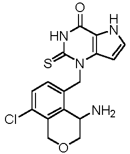 |
| 12 | 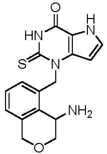 | 13 | 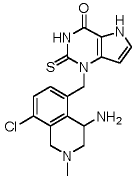 |
| 14 | 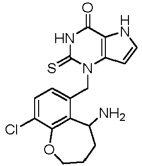 | 15A | 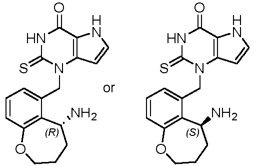 |
| 15B | 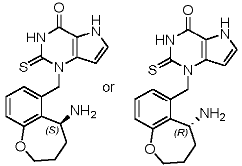 | 16 | 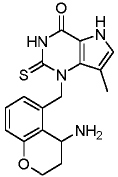 |
| 17 | 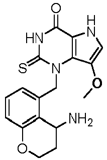 | | |

11. The compound, or the stereoisomer, the tautomer or the pharmaceutically acceptable salt thereof according to claim 1, wherein the pharmaceutically acceptable salt refers to a salt prepared from a compound and a pharmaceutically acceptable acid or base.

12. A pharmaceutical composition, comprising the compound, or the stereoisomer, the tautomer or the pharmaceutically acceptable salt thereof according to any one of claims 1 to 11, and one or more pharmaceutically acceptable carrier.

13. Use of the compound, or the stereoisomer, the tautomer or the pharmaceutically acceptable salt thereof according to any one of claims 1 to 11 in preparation of a drug for treating myeloperoxidase related diseases, preferably in preparation of a drug for treating cardiovascular related diseases.
